(19) Europäisches Patentamt
European Patent Office
Office européen des brevets

(11) **EP 2 040 562 B1**

(12) **EUROPÄISCHE PATENTSCHRIFT**

(45) Veröffentlichungstag und Bekanntmachung des Hinweises auf die Patenterteilung:
**28.04.2010 Patentblatt 2010/17**

(21) Anmeldenummer: **07725371.4**

(22) Anmeldetag: **18.05.2007**

(51) Int Cl.:
*A23J 1/00* (2006.01)      *A23L 2/78* (2006.01)
*A23L 2/80* (2006.01)      *C12C 7/14* (2006.01)
*C12G 1/00* (2006.01)

(86) Internationale Anmeldenummer:
**PCT/EP2007/004463**

(87) Internationale Veröffentlichungsnummer:
**WO 2007/134816 (29.11.2007 Gazette 2007/48)**

(54) **VERFAHREN ZUR ABTRENNUNG VON PROTEINEN AUS FLÜSSIGEN MEDIEN UNTER VERWENDUNG THERMISCH MODIFIZIERTER TONMATERIALIEN**

PROCESS FOR SEPARATING PROTEINS FROM LIQUID MEDIA USING THERMALLY MODIFIED CLAY MATERIALS

PROCÉDÉ DE SÉPARATION DE PROTÉINES DE MILIEUX LIQUIDES PAR UTILISATION DE MATÉRIAUX ARGILEUX MODIFIÉS THERMIQUEMENT

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **20.05.2006 DE 102006023822**

(43) Veröffentlichungstag der Anmeldung:
**01.04.2009 Patentblatt 2009/14**

(73) Patentinhaber: **Süd-Chemie AG**
**80333 München (DE)**

(72) Erfinder:
• **SOHLING, Ulrich**
**85356 Freising (DE)**
• **GERLEIGNER, Alfred**
**80939 München (DE)**
• **STÜTZER, Albert**
**85456 Wartenberg (DE)**
• **RIECHERS, Daniel**
**30451 Hannover (DE)**
• **KASPER, Cornelia**
**30159 Hannover (DE)**
• **SCHEPER, Thomas**
**30559 Hannover (DE)**

(74) Vertreter: **Rembold, Hansjörg**
**Stolmár Scheele & Partner**
**Blumenstraße 17**
**80331 München (DE)**

(56) Entgegenhaltungen:
EP-A- 0 889 004          WO-A-2006/097326
WO-A-2007/073893      DE-A1- 3 633 324
DE-B- 1 160 812          US-A- 5 869 415

• DATABASE CAPLUS [Online] CHEMICAL ABSTRACTS SERVICE, COLUMBUS, OHIO, US; XP002459231 gefunden im STN Database accession no. 2003:789300 & RU 2 209 824 A (OBSHECHESTVO S OGRANICHENNOI OTVESTSTVENNOST'YU) 10. August 2003 (2003-08-10)

**Beschreibung**

Die Erfindung betrifft ein Verfahren zur Abtrennung von Proteinen aus flüssigen Medien.

Die Zellkulturtechnik ermöglicht die Herstellung biologisch aktiver Substanzen im großen Maßstab. Mit derartigen Verfahren können beispielsweise Enzyme für technische Anwendungen hergestellt werden, wie z.B. für die Herstellung von Nahrungsmitteln oder als Bestandteil von Waschmitteln oder auch rekombinante Proteine für pharmazeutische Anwendungen produziert werden. Die gewünschten Proteine werden von Mikroorganismen oder Säugetierzellen produziert und anschließend aus dem Kultivierungsmedium oder den Zellen isoliert. Dieser Herstellungsprozess gliedert sich in die Fermentation mit vorgelagerter Medienaufbereitung (upstream-processing) und in eine nachfolgende Prozesskette, welche zur Reindarstellung des Proteins dient (downstreamprocessing). Die Kosten für die Reinigung des Proteins übertreffen häufig einen Anteil von 80 % der Gesamtherstellungskosten. Zur Aufreinigung werden meist chromatographische Methoden verwendet, die einerseits zur Anreicherung des Proteins im Prozessstrom, andererseits zur Abtrennung von Fremdproteinen und anderen Kontaminationen, wie Endotoxinen, Viren oder DNA dienen. Die Chromatographiematerialien müssen bei mehrfacher Verwendung während ihrer Lebenszeit ständig auf ihre Trennleistung oder eine eventuelle mikrobielle Kontamination überprüft werden. Durch die Verwendung von Einmal-Materialien könnten die Kosten für Validierung und Reinigung zumindest teilweise eingespart werden. Für einen erfolgreichen Einsatz muss ein Einmalchromatographiematerial eine reproduzierbar hohe Trennleistung erbringen und einen niedrigen Investitionsaufwand pro Verbrauchseinheit erfordern. Es sollte hohe Bindungskapazitäten und eine schnelle Bindungskinetik für die interessierenden Zielproteine oder die störenden Kontaminationen aufweisen.

Ein weiteres technisches Gebiet, bei welchem Proteine in größerem Maßstab aus flüssigen Medien entfernt werden, betrifft die Herstellung von Getränken, wie Wein, Bier oder klaren Fruchtsäften. Zur Stabilisierung und Verbesserung der optischen Erscheinung werden Proteine, welche noch in den Getränken enthalten sind, entfernt. Die Proteine wirken als Störstoffe, die bei einer längeren Lagerung beispielsweise ausfallen können oder durch Zersetzung zu einer Beeinträchtigung der Qualität des Lebensmittels führen. Es müssen bei diesem Reinigungsprozess relativ große Flüssigkeitsvolumen verarbeitet werden, um geringe Mengen an Proteinen abzutrennen.

Bentonite und insbesondere deren mineralische Hauptkomponente Montmorillonit können Proteine reversibel binden. Diese Mineralien wirken als natürliche Kationenaustauscher und können Proteine bei pH-Werten unterhalb Ihres isoelektrischen Punktes adsorbieren. Da sie aus natürlichen Quellen abgebaut werden und keine aufwändige Aufbereitung benötigen, stehen sie an sich kostengünstig und in großen Mengen zur Verfügung.

Über die Verwendung von Schichtsilicaten zur Abtrennung von Enzymen aus dem filtrierten Saft homogenisierter Zuckerrüben berichten C. Buttersack, K. Nowikow, A. Schaper und K. Buchholz (Zuckerind. 119 (1994) Nr. 4, S. 284-291). Durch Adsorption an Natriumbentonit und anschließende Desorption mit einem pH-Puffer konnten auf einfache Weise Enzyme in reiner Form abgetrennt werden. Die Arbeiten zeigen die gute Eignung von Schichtmineralien zur Abtrennung bzw. Anreicherung von Proteinen. Die Handhabung der Schichtmineralien ist jedoch kompliziert, da diese in Wasser zu einer starken Erhöhung der Viskosität der Aufschlämmung führen und die Schichtmineralien in sehr kleine Partikel zerfallen. Es ist daher sehr schwierig, die Anreicherung von Proteinen in der Weise durchzuführen, dass das Schichtmineral in eine Säule gepackt wird, über welche dann die das Protein enthaltende Flüssigkeit geleitet wird. Ohne Hilfsmaßnahmen verstopft die Säule relativ rasch, da das Schichtmineral stark quillt. Um dennoch eine Abtrennung der Enzyme in einer Säule zu ermöglichen, schlagen die Autoren vor, den Natriumbentonit in ein Calciumalginatgel einzugießen. Das hydrierte Homogenat wird bei pH = 5,0 über die Säule gepumpt, die mit Perlen des immobilisierten Bentonits gefüllt ist. Nachdem die nicht-adsorbierte Fraktion aus der Säule ausgetreten ist, wird mit einer auf pH = 8 eingestellten Pufferlösung das Enzym eluiert. Für die großtechnische Abtrennung von Proteinen ist die Verwendung von Natriumbentonit, welcher wie von den Autoren beschrieben, in einem Calciumalginat fixiert ist, wegen der hohen anfallenden Kosten wenig geeignet.

In der DE 1 160 812 wird ein Verfahren zur Erhöhung der Eiweißstabilität von Bier beschrieben. Dazu wird das Bier mit einem feinpulvrigen, weitgehend dehydratisierten und damit lagerfähigen Kieselsäurexerogel behandelt, das eine innere Oberfläche von 200 bis 400 m$^2$/g, ein Porenvolumen von mehr als 0,6 ml/g und einen Porendurchmesser von mehr als 60 Ångström aufweist. Um die Anforderungen des Reinheitsgebotes zu erfüllen, wird das Kieselgel sorgfältig gewaschen, so dass der Anteil an wasserlöslichen Stoffen bis auf unter 1 % abgesenkt wird.

In der DE 1 717 084 A wird ein Verfahren zur Erhöhung der Eiweißstabilität zur Verbesserung der biologischen Haltbarkeit von Bier beschrieben. Dazu wird das Bier mit feinstgemahlenem weitbis mittelporigem Kieselgel behandelt, das nach ausreichender Einwirkzeit wieder vom Bier abgetrennt wird. Das feinstgemahlene weit- bis mittelporige Kieselgel soll zu 75 bis 90 Gew.-% eine Teilchengröße unter 44 μm aufweisen.

In der DE 1 717 085 B wird ein Adsorptionsmittel für die Behandlung gegorener Getränke, insbesondere Bier, beschrieben. Das Adsorptionsmittel beruht auf säureaktivierten silicatischen Schichtmineralien mit quellbarem Gitter. Bevorzugt werden Montmorin-Mineralien, z.B. Montmorillonit und Hektorit eingesetzt, die im gequollenen Zustand einer Säureaktivierung unterworfen wurden und deren Säureaufschluss unter Erhaltung der gequollenen Gitterstruktur gewaschen und abgepresst worden ist. Das Adsorptionsmittel besitzt ein Porenvolumen von 0,3 bis 0,8, vorzugsweise 0,4 bis 0,7 ml/g, festgestellt an einer Probe, die für die Analyse bei 130 ˚C getrocknet wurde. Die Herstellung des Adsorptionsmittels erfolgt durch Extrahieren der silicatischen Schichtmineralien mit siedender Mineralsäure, wobei Menge und Konzentration der Mineralsäure, Erhitzungsdauer, Temperatur und Druck so gewählt werden, dass das gewünschte Porenvolumen eingestellt wird.

In der DE-OS-15 17 888 wird ein Adsorptionsmittel für die Behandlung gegorener Getränke, insbesondere Bier, beschrieben. Wie bei der DE 1 717 085 B wird ein mit Säure aufgeschlossenes silicatisches Schichtmineral verwendet, welches ein Porenvolumen von 0,3 bis 0,8, vorzugsweise 0,4 bis 0,7 ml/g aufweist, festgestellt an einer Probe, die für die Analyse bei 130 ˚C getrocknet wurde. Das aufgeschlossene Schichtmineral wird vor der Anwendung bis zu einem Feststoffgehalt von bis zu 80 Gew.-% partiell getrocknet. Gemäß einer bevorzugten Ausführungsform des Adsorptionsmittels wird diesem eine Wasserglaslösung zugegeben, so dass es einen Anteil an Kieselsäurehydrat besitzt. Durch den Zusatz von Wasserglas modifiziertes Adsorptionsmittel lässt sich leichter und feiner zerkleinern und als Folge in Flüssigkeiten besonders leicht verteilen. Dadurch wirkt es besonders fördernd auf die Klärung von beispielsweise Bier.

In der DE 1 642 767 B wird die Verwendung eines Adsorptionsmittels für die Klärung gegorener Getränke, wie Bier oder Wein, beschrieben, wobei das Adsorptionsmittel aus sauer aktivierten Montmorin-Mineralien hergestellt wird, die nach einem Säureaufschluss mit wässrigen oder sauren Lösungen von polymeren Phosphaten oder Polyphosphorsäuren behandelt worden sind.

Schichtmineralien zeigen potentiell eine hohe Bindungskapazität für Eiweiß und können relativ kostengünstig zur Verfügung gestellt werden. Sie stellen daher ein sehr attraktives Mittel zur Schönung von beispielsweise Getränken, wie Wein oder Bier, oder zur Abtrennung wertvoller Proteinen bei der biotechnologischen Herstellung dar. Allerdings stehen einer Anwendung die oben geschilderten Probleme im Wege. Die Abtrennung von Proteinen mit Hilfe von Schichtmineralien lässt sich nur unter großem Aufwand und mit Hilfe von chromatographischen Säulen durchführen. Wird das Schichtmineral direkt dem zu verarbeitenden wässrigen Medium zugegeben, lässt es sich nur schwierig durch Filtration abtrennen, da durch die Feinteiligkeit der Partikel ein Filter relativ rasch verstopft.

Der Erfindung lag die Aufgabe zugrunde, ein Verfahren bereitzustellen, mit welchem auch in großtechnischem Maßstab eine Abtrennung von Proteinen aus flüssigen Medien gelingt, wobei das Verfahren nach Möglichkeit kontinuierlich durchführbar sein soll.

Die Aufgabe wird mit einem Verfahren mit den Merkmalen des Patentanspruchs 1 gelöst. Vorteilhafte Weiterbildungen des erfindungsgemäßen Verfahrens sind Gegenstand der abhängigen Ansprüche.

Bei dem erfindungsgemäßen Verfahren zur Abtrennung von Proteinen aus flüssigen Medien wird:

- ein proteinhaltiges flüssiges Medium bereitgestellt,

- das proteinhaltige flüssige Medium zur Abreicherung an Proteinen mit einem hitzebehandelten Tonmineral in Kontakt gebracht, das eine Quellfähigkeit in Wasser nach einer Stunde von weniger als 15 ml/2 g aufweist und

- das an Proteinen abgereicherte flüssige Medium von dem Tonmaterial abgetrennt.

Beim erfindungsgemäßen Verfahren wird ein hitzebehandeltes Tonmaterial als Adsorptionsmittel für Proteine verwendet. Durch die Hitzebehandlung verliert das Tonmaterial seine Quellfähigkeit weitgehend. Die Hitzebehandlung wird in der Weise durchgeführt, dass die Quellfähigkeit des hitzebehandelten Tonmaterials in Wasser weniger als 15 ml/2 g, vorzugsweise weniger als 10 ml/2 g beträgt und damit im Wesentlichen dem Sedimentvolumen entspricht. Das Tonmaterial kann wegen seiner geringen Quellfähigkeit problemlos in größeren Mengen zu einem flüssigen, insbesondere wässrigen Medium gegeben werden, ohne dass dadurch beispielsweise die Viskosität der Suspension drastisch ansteigt. Ferner lässt es sich ohne Schwierigkeiten mit üblichen Verfahren, wie Filtration oder Zentrifugation, wieder vom flüssigen Medium abtrennen. Die Proteine bleiben dabei an dem hitzebehandelten Tonmaterial absorbiert, so dass das flüssige Medium entsprechend an Proteinen abgereichert wird.

Überraschenderweise wurde gefunden, dass sich durch Hitzebehandlung ein Tonmaterial erhalten lässt, das Proteine in solchen Mengen binden kann, welche für eine technische Anwendung interessant sind. An sich geht man nach herrschender Meinung bei Schichtsilicaten davon aus, dass bei einem vollständigen Verlust des Quellvermögens der Tonmaterialien die für eine Adsorption zur Verfügung stehende innere Oberfläche stark verringert wird, da die Schichten chemisch miteinander verbunden werden. Man erwartet daher, dass durch eine Hitzebehandlung das Adsorptionsvermögen der Tonmaterialien drastisch abgesenkt wird. Überraschenderweise wurde nun gefunden, dass durch eine Temperaturbehandlung das Quellvermögen von Tonmaterialien, insbesondere Bentoniten, zerstört werden kann, wobei trotzdem das hohe Bindevermögen für Proteine erhalten bleibt.

Als proteinhaltiges flüssiges Medium wird vorzugsweise ein wässriges Medium verwendet, wie beispielsweise ein Getränk, wie Bier, Wein oder Fruchtsäfte, oder ein Kulturmedium, wie es für biotechnologische Prozesse verwendet wird. Das flüssige Medium kann zunächst durch eine Grobfiltration von Schwebstoffen befreit werden. Dies ist insbesondere dann bevorzugt, wenn das hitzebehandelte Tonmaterial beispielsweise als Filterpackung in einem kontinuierlichen Prozess verwendet wird, da dann die Lebensdauer der Filterpackung oder einer entsprechenden Filterpatrone verlängert werden kann. Das flüssige Medium enthält Proteine, wobei hier an sich keine Beschränkungen beispielsweise im Hinblick auf das Molekülgewicht der Proteine bestehen. Bevorzugt liegen die Proteine im flüssigen Medium in gelöster Form vor. Die Proteine weisen dann eine Ladung auf, welche eine Bindung der Proteine an das hitzebehandelte Tonmaterial durch Kationenaustausch ermöglicht. Bevorzugt wird das flüssige Medium daher auf einen pH-Wert eingestellt, der um zumindest eine pH-Einheit unterhalb des isoelektrischen Punktes des interessierenden Proteins liegt. Dazu kann dem flüssigen Medium ein entsprechender Puffer zugegeben werden. Die Auswahl des geeigneten Puffers kann vom Fachmann in der Weise vorgenommen werden, dass zunächst der isoelektrische Punkt für das proteinhaltige flüssige Medium bestimmt wird (pI) und dann ein Puffer ausgewählt wird, dessen Pufferbereich (bzw. pKs-Wert) etwa eine Einheit unterhalb dieses Wertes liegt. Geeignete Puffer sind beispielsweise Citratpuffer oder Phosphatpuffer. Die Konzentration des Puffers wird im üblichen Bereich gewählt. Geeignete Konzentrationen sind beispielsweise 20 bis 100 mmol, bevorzugt etwa 50 mmol.

Die Behandlung des proteinhaltigen flüssigen Mediums mit dem hitzebehandelten Tonmaterial wird bei einer Temperatur durchgeführt, bei welcher die Proteine nicht denaturiert werden. Bevorzugt wird das Verfahren bei Raumtemperatur oder Temperaturen unterhalb der Raumtemperatur, bevorzugt in einem Bereich von etwa 0 bis 20 ˚C durchgeführt.

Nachdem die zu reinigende Probe gegebenenfalls durch Filtration, Kühlung oder Zugabe eines geeigneten Puffers vorbereitet worden ist, wird das hitzebehandelte Tonmaterial zugegeben. Die Zugabe kann an sich in beliebiger Weise erfolgen. So kann das Tonmaterial in trockener Form, beispielsweise als Pulver, zugegeben werden. Es ist aber auch möglich das Tonmaterial zuvor in Wasser oder einem geeigneten Puffer zu suspendieren und anschließend die Suspension zum proteinhaltigen flüssigen Medium zuzugeben. Ferner ist es auch möglich, das hitzebehandelte Tonmaterial als stationäre Phase zu verwenden, beispielsweise in Form einer Filterpackung oder einer Chromatographiesäule, und das proteinhaltige flüssige Medium durch eine Schicht des hitzebehandelten Tonmaterials durchzuleiten. Die Menge des zu verwendenden hitzebehandelten Tonmaterials ist von der Menge des zu reinigenden proteinhaltigen flüssigen Mediums abhängig, sowie von der Konzentration an Proteinen im flüssigen Medium. Ferner kann das proteinhaltige flüssige Medium auch noch andere geladene Verbindungen enthalten, beispielsweise Zuckersäuren oder Phosphate, die ebenfalls an das hitzebehandelte Tonmaterial gebunden werden können. Die geeignete Menge an hitzebehandeltem Tonmaterial kann fallabhängig vom Fachmann durch entsprechende Reihenversuche bestimmt werden.

Das hitzebehandelte Tonmaterial wird dann für einen bestimmten Zeitraum in Kontakt mit dem proteinhaltigen flüssigen Medium gebracht. Es wurde beobachtet, dass die Bindung der Proteine an das hitzebehandelte Tonmaterial relativ rasch erfolgt, so dass keine übermäßig langen Kontaktzeiten eingehalten werden müssen. Die geeigneten Kontaktzeiten sind von der Art und Menge der abzutrennenden Proteine sowie von der Menge des zur Verfügung stehenden hitzebehandelten Tonmaterials abhängig und können vom Fachmann durch entsprechende Reihenversuche ermittelt werden. Bei Verwendung einer Filterpackung oder einer Chromatographiesäule kann beispielsweise der Zeitpunkt überwacht werden, zu welchem die Konzentration an Protein im Eluat ansteigt. Wird das hitzebehandelte Tonmaterial zu einem proteinhaltigen flüssigen Medium zugegeben, kann die Proteinkonzentration im Überstand herangezogen werden, um die Adsorptionsmenge zur überwachen.

Abschließend wird das an Protein abgereicherte flüssige Medium von dem Tonmaterial abgetrennt. Dazu können, wie bereits oben erläutert, übliche Verfahren verwendet werden, beispielsweise Filtration oder Zentrifugation. Bei Verwendung des Tonmaterials als stationäre Phase umfasst das Eluat zwangsläufig kein Tonmaterial.

Die Eigenschaften des hitzebehandelten Tonmaterials lassen sich durch die Temperaturbehandlung steuern. Da das Rohtonmaterial bevorzugt aus natürlichen Quellen gewonnen wird und daher Schwankungen in den Eigenschaften der verschiedenen Rohtonmaterialien auftreten können, wird bevorzugt in der Weise vorgegangen, dass zunächst durch Reihenuntersuchungen die optimale Temperatur für den Rohton ermittelt wird, bei welchem die gewünschte niedrige Quellfähigkeit eingestellt wird. Bevorzugt wird dabei eine möglichst niedrige Temperatur gewählt und gegebenenfalls die Dauer der Temperaturbehandlung angepasst. Bevorzugt werden für die Temperaturbehandlung Temperaturen von zumindest 400 ˚C, bevorzugt zumindest 450 ˚C, insbesondere bevorzugt zumindest 500 ˚C gewählt. Das Rohtonmaterial sollte jedoch auch keine zu hohe thermische Belastung erfahren, da sonst die Adsorptionskapazität für Proteine absinkt. Bevorzugt werden die Temperaturen daher niedriger als 1000 ˚C, vorzugsweise niedriger als 800 ˚C und insbesondere bevorzugt unterhalb von 700 ˚C gewählt. Die Dauer der Behandlung ist abhängig von der Menge des eingesetzten Rohtonmaterials sowie vom Ofentyp. Bevorzugt werden Drehrohrofen für die Hitzebehandlung verwendet, da diese ein gleichmäßiges Erhitzen des Rohtonmaterials ermöglichen. Die Behandlungsdauer wird vorzugsweise möglichst kurz gewählt, um eine übermäßige thermische Belastung des Rohtonmaterials zu vermeiden. Bei einer industriellen Durchführung des Verfahrens werden Behandlungszeiten von zumindest 10 Minuten, vorzugsweise zumindest 20 Minuten gewählt. Um eine thermische Überbelastung zu vermeiden, wird die Behandlungszeit bevorzugt kürzer als 2 Stunden, insbesondere bevorzugt kürzer als 1 Stunde gewählt. Die geeignete Behandlungsdauer kann vom Fachmann leicht ermittelt werden, indem während der thermischen Behandlung des Rohtons regelmäßig Proben entnommen und auf ihre Quellfähigkeit untersucht werden. Weitere Parameter, wie die Ionenaustauschkapazität oder das Porenvolumen, können für die Überwachung der thermischen Behandlung ebenfalls unterstützend herangezogen werden.

Als Ausgangsmaterial für die Herstellung des thermisch behandelten Tonmaterials können an sich beliebige Rohtonmaterialen verwendet werden, die aus natürlichen Quellen zugänglich sind. Bevorzugt werden Rohtonmaterilien verwendet, die eine hohe Ionenaustauschkapazität sowie eine hohe Quellfähigkeit aufweisen. Bevorzugt werden Tonmaterilien verwendet, die eine Ionenaustauschkapazität von zumindest 10 meq/100 g, vorzugsweise zumindest 20 meq/100 g, insbesondere bevorzugt zumindest 40 meq/100 g, besonders bevorzugt im Bereich von 50 bis 130 meq/100 g, insbesondere bevorzugt im Bereich von 70 bis 120 meq/100 g aufweisen. Ferner werden bevorzugt Tonmaterialien verwendet, die eine hohe Quellfähigkeit aufweisen. Bevorzugt weisen die als Ausgangsmaterial verwendeten Tonmaterialen eine Quellfähigkeit in Wasser nach einer Stunde von zumindest 10 ml/2 g, vorzugsweise zumindest 15 ml/2 g, besonders bevorzugt zumindest 20 ml/2 g, insbesondere bevorzugt im Bereich von 30 bis 80 ml/2 g auf. Geeignet sind beispielsweise Natriumbentonite oder Calciumbentonite.

Durch die thermische Behandlung wird die Quellfähigkeit des Rohtonmaterials bevorzugt um zumindest 5 %, besonders bevorzugt um zumindest 10 %, insbesondere bevorzugt um zumindest 20 % herabgesetzt. Werden als Rohtonmaterial Tone verwendet, die einen hohen Anteil an zweiwertigen Kationen in den Zwischenschichten enthalten, beispielsweise Calciumbentonite, so zeigen diese bereits eine geringere Quellfähigkeit als Tone, die Alkalimetallionen in den Zwischenschichten enthalten, wie beispielsweise Natriumbentonite. Enthält das Rohtonmaterial einen hohen Anteil an zweiwertigen Kationen, sinkt die Quellfähigkeit bei der thermischen Behandlung daher weniger stark als bei Rohtonmaterialien, die einen hohen Anteil an einwertigen Kationen, insbesondere Alkalimetallionen, enthalten.

Die für die Herstellung der hitzebehandelten Tonmaterialien verwendeten Rohtonmaterialien weisen bevorzugt neutrale bis schwach basische Eigenschaften auf. Eine 2 Gew.-%ige Suspension des Rohtonmaterials in destilliertem Wasser weist vorzugsweise einen pH-Wert im Bereich von 6 bis 11 auf.

Als Ausgangsmaterialien für die Herstellung der hitzebehandelten Tonmaterialien werden bevorzugt smektitische Schichtsilikate verwendet. Hierunter fallen geladene Tonminerale vom 2 : 1 Schichttyp mit einer negativen Ladung von 0,2 bis 0,6 pro Formeleinheit.

Die Kationenumtauschkapazität der als Ausgangsmaterialien für die Herstellung der hitzebehandelten Tonmaterialien verwendeten Rohtonmaterialien lässt sich durch Austausch mit Ammoniumchlorid und anschließende Stickstoffbestimmung ermitteln und liegt vorzugsweise im oben beschriebenen Bereich von 10 bis 130 meq/100 g. Beispiele für geeignete smektitische Tonminerale sind Bentonit, Montmorillonit, Beidellit, Nontronit, Hectorit und Saponit. Ein weiteres geeignetes smektitisches Tonmineral ist Stevensit. Seine Struktur leitet sich vom Talk ab, wobei die Positionen der Magnesiumionen durch Leerstellen ersetzt sind.

Die für die Herstellung des hitzebehandelten Tonmaterials verwendeten Rohtonmaterialien können vor der Hitzebehandlung ggf. getrocknet werden, vorzugsweise auf einen Feuchtegehalt von weniger als 20 Gew.-%, insbesondere auf einen Feuchtegehalt im Bereich von 5 - 15 Gew.-%. Ferner kann das Rohtonmaterial vor der Hitzebehandlung vermahlen werden, vorzugsweise auf eine mittlere Korngröße $D_{50}$ von weniger als 3 mm, besonders bevorzugt weniger als 1 mm, insbesondere bevorzugt weniger als 0,2 mm

Eine hohe Ionenaustauschkapazität korreliert im Allgemeinen mit einer guten Bindekapazität für Proteine. Beim im erfindungsgemäßen Verfahren verwendeten hitzebehandelten Tonmaterial beträgt die Kationenaustauschkapazität vorzugsweise zumindest 15 meq/100 g. Bevorzugt weist das hitzebehandelte Tonmaterial eine möglichst hohe Ionenaustauschkapazität auf. Bevorzugt weist das hitzebehandelte Tonmaterial eine Kationenaustauschkapazität von vorzugsweise zumindest 40 meq/100 g, insbesondere bevorzugt zumindest 60 meq/100 g auf. Besonders bevorzugt liegt die Kationenaustauschkapazität im Bereich von 65 bis 130 meq/100 g. Neben der Quellfähigkeit des hitzebehandelten Tonmaterials kann daher die Ionenaustauschkapazität als weiterer Indikator gewählt werden, um die optimale Behandlungstemperatur sowie Behandlungsdauer des Rohtonmaterials zu ermitteln. Durch die Temperaturbehandlung kann beim hitzebehandelten Tonmaterial im Vergleich zum Ausgangsmaterial eine geringfügige Absenkung der Kationenaustauschkapazität eintreten. Die Ionenaustauschfähigkeit ist ein wichtiges Kriterium, um beispielsweise die im erfindungsgemäßen Verfahren verwendeten hitzebehandelten Tonmaterialien von Tonmaterialien zu unterscheiden, wie sie beispielsweise durch Auslaugen mit heißer Säure aus natürlichen Tonmaterialien gewonnen werden können. Letztere zeigen keine oder nur eine geringe Fähigkeit zum Ionenaustausch. Hochaufgeschlossene Bleicherden, die nicht mehr quellen, zeigen typischerweise eine Kationenaustauschkapazität im Bereich von weniger als 30 meq/100 g.

Das im erfindungsgemäßen Verfahren verwendete hitzebehandelte Tonmaterial kann, wie oben beschrieben, ausgehend von an sich beliebigen, vorzugsweise smektitischen Tonmineralien hergestellt werden. Die als Ausgangsmaterialien verwendeten Rohtonmaterialien können sowohl beispielsweise in der Natriumform als auch in der Calcium- oder Magnesiumform vorliegen. Ebenso können auch Mischformen verwendet werden, in welchen einwertige Alkalimetallionen und zweiwertigen Erdalkalimetallionen als Zwischenschichtionen in den Rohtonmaterialien enthalten sind. Der Anteil der ein- bzw. zweiwertigen Kationen kann vor der Hitzebehandlung eingestellt werden, indem das Rohtonmaterial mit einem entsprechenden Alkalimetallsalz, beispielsweise Natriumcarbonat oder Natriumhydrogencarbonat, umgesetzt wird. Dazu kann beispielsweise ein naturfeuchter Calciumbentonit mit einer Sodalösung besprüht oder mit festem Natriumcarbonat verknetet werden. Soll der Anteil an zweiwertigen Erdalkalimetallionen erhöht werden, kann das Rohtonmaterial mit einer geeigneten Erdalkaliverbindung, beispielsweise Calciumchlorid, umgesetzt werden.

Bevorzugt wird das im erfindungsgemäßen Verfahren verwendete hitzebehandelte Tonmaterial aus einem Rohtonmaterial hergestellt, welches auf Zwischenschichtplätzen zumindest einen Anteil an zweiwertigen Erdalkalimetallionen, vorzugsweise Calciumionen, aufweist.

Bei Bentoniten wird an sich eine umso höhere Bindungskapazität für Proteine beobachtet, je höher der Anteil an Alkalimetallionen an den austauschfähigen, in den Zwischenschichten des Bentonits angeordneten Ionen ist. Die hohe Bindungskapazität von Natriumbentoniten wird auf die starke Delaminierung zurückgeführt, die bei der Herstellung einer Suspension in Wasser eintritt. Dadurch erhöht sich die für eine Absorption zur Verfügung stehende Oberfläche stark. Bei Calciumbentoniten besteht ein stärkerer Zusammenhalt der Schichten, wodurch bei der Herstellung einer wässrigen Suspension eine Delaminierung nur in geringerem Maß auftritt und damit auch eine im Vergleich zu Natriumbentoniten geringere Oberfläche für die Absorption zur Verfügung steht.

Beim erfindungsgemäßen Verfahren hat sich jedoch gezeigt, dass eine höhere Absorptionskapazität für Proteine erreicht werden kann, wenn von einem Rohtonmaterial ausgegangen wird, das einen Anteil an Erdalkaliionen auf Zwischenschichtplätzen aufweist.

Bevorzugt beträgt beim hitzebehandelten Tonmaterial der Anteil an zweiwertigen Kationen an der Ionenaustauschkapazität zumindest 15 %, besonders bevorzugt zumindest 30 %, insbesondere bevorzugt zumindest 70 %. Der Anteil der zweiwertigen Kationen, insbesondere Calcium und Magnesium, an der Kationenaustauschkapazität beträgt meist weniger als 95 %, insbesondere weniger als 85 %. Es wurde beobachtet, dass durch einen hohen Anteil an austauschbaren zweiwertigen Kationen die Bindungsneigung von Proteinen an das hitzebehandelte Tonmaterial verbessert werden kann. Ohne an diese Theorie gebunden sein zu wollen, nehmen die Erfinder an, dass die in Zwischenschichten angeordneten Erdalkaliiionen bei der thermischen Behandlung des Rohtonmaterials eine Stützfunktion ausüben, sodass die Schichten des Rohtonmaterials nicht so stark zusammensinken, wie bei reinen Natriumbentoniten. Der Anteil der zwei- bzw. einwertigen Kationen kann entsprechend vor der Hitzebehandlung beim Rohtonmaterial eingestellt werden. Dieses kann dazu beispielsweise mit einem geeigneten Natriumsalz, wie Natriumcarbonat, umgesetzt werden, indem das Rohtonmaterial beispielsweise mit einer Sodalösung besprüht oder mit festem Natriumcarbonat verknetet wird.

Durch die Hitzebehandlung wird die innere Oberfläche des Tonmaterials deutlich verringert. Vorzugsweise weist das hitzebehandelte Tonmaterial eine spezifische Oberfläche von weniger als 300 $m^2$/g, insbesondere bevorzugt weniger als 200 $m^2$/g auf, besonders bevorzugt weniger als 120 $m^2$/g. Um eine ausreichende Absorptionskapazität für Proteine zu erreichen, darf die spezifische Oberfläche allerdings auch nicht zu weit abgesenkt werden, das Tonmaterial also nicht "totgebrannt" werden. Vorzugsweise beträgt die spezifische Oberfläche des hitzebehandelten Tonmaterials zumindest 30 $m^2$/g, insbesondere bevorzugt zumindest 60 $m^2$/g.

Bevorzugt wird die spezifische Oberfläche des Rohtonmaterials durch die Hitzebehandlung um zumindest 5 %, besonders bevorzugt zumindest 10 % erniedrigt.

Gemäß einer besonders bevorzugten Ausführungsform weist das im erfindungsgemäßen Verfahren verwendete hitzebehandelte Tonmaterial nahezu keine Quellfähigkeit in Wasser mehr auf. Das Quellvolumen entspricht in diesem Fall dem Sedimentvolumen. Auch bei einer längeren Lagerung in Wasser ist keine oder nahezu keine Zunahme des Sedimentvolumens zu beobachten. Bevorzugt weist das hitzebehandelte Tonmaterial nach einer Lagerung in Wasser für drei Tage ein Sedimentvolumen von weniger als 15 ml/2 g, bevorzugt weniger als 10 ml/2 g, insbesondere bevorzugt von weniger als 8 ml/2 g auf.

Nach der Hitzebehandlung wird das Tonmaterial bevorzugt keiner weiteren Aktivierung unterzogen. Es kann noch durch physikalische Verfahrensschritte, wie Mahlen oder Sieben, eine gewünschte Korngröße eingestellt werden. Bevorzugt wird das hitzebehandelte Tonmaterial auf eine mittlere Korngröße $D_{50}$ im Bereich von 10 - 200 $\mu$m eingestellt. Durch Granulation können für spezielle Anwendungen auch größere Teilchen erzeugt werden, beispielsweise für Anwendungen, in denen eine geringe Staubbelastung erwünscht ist bzw. sich das Granulat einfacher dosieren lässt. Bevorzugt wird das hitzebehandelte Tonmaterial jedoch keiner Oberflächenaktivierung durch Säure unterworfen.

Um eine Denaturierung der Proteine zu verhindern, sowie um einen geeigneten pH-Wert für eine möglichst effiziente Adsorption der Proteine am hitzebehandelten Tonmaterial bereitzustellen, wird das Tonmaterial vor der Aufgabe des flüssigen Mediums bevorzugt auf einen pH-Wert von etwa 3,5 bis 6,0 equilibriert. Dazu kann das hitzebehandelte Tonmaterial beispielsweise in einem geeigneten Puffermedium aufgeschlämmt werden oder eine Filterpackung bzw. Chromatographiesäule durch Durchleiten eines geeigneten Puffers auf den gewünschten pH-Bereich eingestellt werden.

Gemäß einer bevorzugten Ausführungsform wird das hitzebehandelte Tonmaterial als stationäre Phase verwendet, durch welche das proteinhaltige flüssige Medium durchgeleitet wird. Gemäß einer ersten Ausführungsform wird das flüssige Medium zur Abtrennung der Proteine durch eine Filterpackung geleitet, welche zumindest anteilig aus dem hitzebehandelten Tonmaterial gebildet ist. Die Filterpackung kann dabei an sich jede beliebige Form annehmen. Geeignet sind beispielsweise Filterpatronen, welche mit dem Tonmaterial gefüllt sind. Nachdem die Aufnahmekapazität der Filterpatrone erschöpft ist, kann diese gegen eine neue Patrone ausgetauscht werden. Die mit den Proteinen beladene Filterpatrone kann entweder verworfen werden oder einer Aufarbeitung zugeführt werden, in welcher die auf dem hitzebehandelten Tonmaterial absorbierten Proteine wiedergewonnen werden.

Gemäß einer bevorzugten Ausführungsform wird die Filterpackung durch eine Anschwemmfiltration hergestellt. Das Tonmaterial wird dabei vorzugsweise mit einem Filterhilfsmittel, wie Kieselgur oder Perlit® gemischt. Ferner kann das Tonmaterial auch noch mit einem weiteren Adsorbens, wie Kieselgel oder sauer aktiviertem Bentonit, welcher nicht thermisch vorbehandelt wurde, gemischt sein. Bei der Filtration bildet das hitzebehandelte Tonmaterial zusammen mit dem Filterhilfsmittel und gegebenenfalls weiteren Adsorbentien einen Filterkuchen aus, der das filtrationswirksame Medium für nachfolgende Trübstoff- und Filterhilfsmittelteilchen bildet.

Bevorzugt wird vor der direkten Filtration eine Filterhilfsmittelschicht als Primärschicht auf ein Filtermittel aufgebracht. Als Filtermittel kann beispielsweise ein Sieb mit einer geeignet geringen Maschenweite verwendet werden. Als Filterhilfsmittel zur Ausbildung einer Primärschicht kann beispielsweise Kieselgur verwendet werden. Dieser Prozess wird auch als Voranschwemmung bezeichnet. In der anschließenden Filtrationsphase wird der Suspension weiteres Filterhilfsmittel zugesetzt, das erfindungsgemäß das oben beschriebene Tonmaterial umfasst. Diese Filterhilfsmittelteilchen bilden gemeinsam mit den Trübstoffteilchen einen Filterkuchen aus, der als Sekundärschicht bezeichnet wird.

Durch die Verwendung des Tonmaterials als Filtermaterial kann das erfindungsgemäße Verfahren auch in kontinuierlicher Form durchgeführt werden, was beispielsweise insbesondere für die Klärung von Getränken von Vorteil ist.

Gemäß einer weiteren Ausführungsform des erfindungsgemäßen Verfahrens wird dieses in Form einer Chromatographie durchgeführt. Dabei wird das hitzebehandelte Tonmaterial in eine Chromatographiesäule gepackt und das flüssige Medium, aus welchem die Proteine abgetrennt werden sollen, auf die Säule gegeben. Die Säule kann von einem Elutionsmittel durchströmt werden, wobei durch Adsorptionseffekte eine Auftrennung der Proteine in unterschiedliche Fraktionen möglich ist. Dies ist insbesondere interessant für die Abtrennung von Proteinen, welche in Bioreaktoren erzeugt wurden.

Die Eluierung der Proteine kann nach verschiedenen Verfahren erfolgen. Beispielsweise kann ein Salzgradient über die Säule geleitet werden, sodass die Proteine bei unterschiedlicher Salzkonzentration eluiert werden. Proteine werden u.a. durch elektrische Wechselwirkungen und/oder van-der-Waals-Kräfte an das im erfindungsgemäßen Verfahren verwendete hitzebehandelte Tonmaterial gebunden. Bei der Eluierung mit einem Puffer mit hoher Salzkonzentration werden die elektrostatischen Wechselwirkungen zwischen dem hitzebehandelten Tonmaterial, welches als Kationenaustauscher wirkt, und dem Protein herabgesetzt. Das Protein kann daher vom Tonmaterial abgelöst und eluiert werden.

Nach einer weiteren Ausführungsform erfolgt die Eluierung der gebundenen Proteine durch eine Änderung des pH-Wertes. Dabei weist das Elutionsmittel einen zum flüssigen Medium unterschiedlichen pH auf.

Beispielsweise können Proteine bei einem pH-Wert aufgegeben werden, bei welchem diese in geladener Form vorliegen. Der pH-Wert des flüssigen Mediums wird dabei vorzugsweise so eingestellt, dass er unterhalb des isoelektrischen Punktes des Proteins liegt, das Protein also eine positive Gesamtladung aufweist. Durch die Kationenaustauscherwirkung des hitzebehandelten Tonmaterials werden die Proteine gebunden. Das im erfindungsgemäßen Verfahren verwendete hitzebehandelte Tonmaterial wirkt also als Kationenaustauschermaterial. Wird nun der pH soweit angehoben, dass der isoelektrische Punkt des Proteins erreicht oder überschritten wird, weist das Protein in den meisten Fällen eine negative Gesamtladung auf. Das Protein wird daher wieder vom Tonmaterial bzw. der Filterschicht abgelöst und kann eluiert werden.

Gegebenenfalls kann dem Elutionsmittel Glycerin beigegeben sein. Glycerin weist eine sehr hohe Affinität zum hitzebehandelten Tonmaterial auf und kann daher die Ablösung des Proteins bzw. der organischen Verbindungen vom Tonmaterial erleichtern.

Das flüssige Medium umfasst bevorzugt Wasser als Lösungsmittel.

Das im erfindungsgemäßen Verfahren verwendete Tonmaterial kann allein oder auch in Kombination mit einem weiteren Adsorptionsmittel verwendet werden. Das weitere Adsorptionsmaterial ist vorzugsweise ausgewählt aus der Gruppe, die gebildet ist aus Kieselgel, Zellulose und Polyvinylpyrolidon.

Das hitzebehandelte Tonmaterial und das weitere Adsorptionsmittel liegen bevorzugt in einem Gewichtsverhältnis zwischen 1:10 und 10:1 vor. In Abhängigkeit von der beabsichtigten Anwendung können jedoch auch Verhältnisse außerhalb des angegebenen Bereichs zur Anwendung gelangen.

In der Bierfiltration werden beispielsweise Xerogele verwendet, die eine hohe spezifische Oberfläche und einen geringen Wassergehalt aufweisen. Der Wassergehalt dieser Xerogele liegt im Bereich von etwa 10 bis 15 %. Diese Xerogele werden vor allem in geschlossenen Systemen verwendet. Wegen der Staubentwicklung werden bei offenen Systemen bevorzugt Semihydrogele mit einem Wassergehalt im Bereich von etwa 30 bis 40 Gew.-% oder Hydrogele mit einem Wassergehalt von mehr als 55 Gew.-% verwendet. Das im erfindungsgemäßen Verfahren verwendete Tonmaterial eignet sich wegen seiner hohen Wasseraufnahmekapazität besonders für die Herstellung von Mischungen mit Semihydrogelen oder Hydrogelen, wobei die Mischung dann im erfindungsgemäßen Verfahren eingesetzt werden kann.

Zur Herstellung derartigere Mischungen kann das Tonmaterial beispielsweise in einem Intensivmischer vorgelegt und dann mit der entsprechenden Menge an Wasser beaufschlagt werden. Bei einer Beaufschlagung mit einer Wassermenge von beispielsweise 50 Gew.-% wird ein fließfähiges Pulver erhalten, welches alleine oder in Mischung mit anderen Adsorptionsmitteln, insbesondere Semihydrogelen und Hydrogelen, im erfindungsgemäßen Verfahren eingesetzt werden kann.

Das erfindungsgemäße Verfahren eignet sich besonders für die Filtration von Getränken, insbesondere Bier. Dabei wird vorzugsweise das Bier oder ein anderes Getränk mit dem Tonmaterial behandelt, indem das hitzebehandelte Tonmaterial zu dem Bier gegeben wird, sodass eine Suspension erhalten wird. Dem hitzebehandelten Tonmaterial kann dabei gegebenenfalls Kieselgel als weiteres Adsorptionsmittel beigegeben sein. Das Tonmaterial wird anschließend vorzugsweise durch eine Anschwemm-/Kuchenfiltration aus der Suspension abgetrennt.

In der Brauerei geht man bei der Bierherstellung dabei vorteilhaft wie folgt vor: Auf einem Metallsieb oder einer Filterschicht aus Zellulose wird zunächst eine Voranschwemmung mit Kieselgur durchgeführt. Hierzu wird Kieselgur in Wasser suspendiert und die Suspension durch das Metallsieb bzw. die Filterschicht gepumpt, so dass eine dünne Kieselgurschicht erhalten wird. Dies dient dazu, ein Durchbrechen der Mischung aus Tonmaterial und Kieselgur zu verhindern. Im allgemeinen werden für die Filtration des Bieres Siebe mit einer Maschenweite im Bereich von etwa 45 $\mu$m verwendet. In das Bier wird das Tonmaterial, gegebenenfalls in Mischung mit Kieselgur, eingerührt und die erhaltene Biersuspension durch den mit einer Voranschwemmschicht bedeckten Filter gepumpt. In der sich aus dem Tonmaterial und gegebenenfalls Kieselgur ausbildenden Schicht werden dann Trübstoffe, wie Proteine oder Protein-Polyphenolkomplexe, in adsorbierter Form zurückgehalten. Der Filtriervorgang wird auch als Kuchenfiltration bezeichnet.

Als weiteres beispielhaftes Anwendungsgebiet für das erfindungsgemäße Verfahren kann dieses zur Abwasserreinigung verwendet werden, insbesondere zur Abtrennung von Proteinen oder Mikroorganismen. Das Abwasser kann dazu, gegebenenfalls nach einer Vorreinigung, über eine Filterpackung geleitet werden, welche aus dem im erfindungsgemäßen Verfahren verwendeten hitzebehandelten Tonmaterial besteht, bzw. dieses enthält. Dies ermöglicht eine einfache und kostengünstige Verringerung der Fracht des Abwassers an organischen Verunreinigungen. Beispielhafte Abwässer, die mit dem erfindungsgemäßen Verfahren gereinigt werden können, sind Abwässer, wie sie bei Fermentationsprozessen oder in Brauereien anfallen.

Das erfindungsgemäße Verfahren wird anhand der folgenden Beispiele sowie unter Bezugnahme auf die beigefügten Figuren näher erläutert. Dabei zeigt:

Figur 1: eine Grafik, in welcher die nach der Eiweißentfernung bestimmte Trübung gegen die Menge an eingesetztem Adsorptionsmittel (Bentonit 1) aufgetragen ist;

Figur 2: eine Grafik, in welcher die nach der Eiweißentfernung bestimmte Trübung gegen die Menge an eingesetztem Adsorptionsmittel (Bentonit 3) aufgetragen ist,;

Figur 3: eine Grafik, in welcher die pH-Abhängigkeit der Adsorption von Modellproteinen an ein thermisch behandeltes Tonmaterial dargestellt ist;

Figur 4: eine Grafik, in welcher die Adsorptionsisothermen verschiedener Modellproteine an ein hitzebehandeltes Tonmaterial in einem statischen System dargestellt sind; dabei zeigt

Figur 4a: eine Adsorptionsisotherme von alkalischer Phosphatase an Bentonit $1_{500}$ (50 mM Acetat-Puffer pH 5; 3 h Inkubation);

Figur 4b: eine Adsorptionsisotherme von $\alpha$-Chymotrypsin an Bentonit $1_{500}$ (50 mM Tris-Puffer pH 8; 3 h Inkubation)

Figur 4c: eine Adsorptionsisotherme von humanem Serum Albumin an Bentonit $1_{500}$ (50 mM Citrat-Puffer pH 4; 3 h Inkubation);

Figur 5: eine Grafik, in welcher die Adsorptionsisothermen verschiedener Modellproteine an ein hitzebehandeltes Tonmaterial in einem dynamischen System dargestellt sind; dabei zeigt:

Figur 5a: die Beladung von Bentonit $1_{500}$ (50 mM Acetat-Puffer, pH 5) mit alkalischer Phosphatase ($c_0$(AP) =1 mg·ml$^{-1}$) in Abhängigkeit von der Auftragungsgeschwindigkeit;

Figur 5b: die Beladung von Bentonit 1500 (50mM Tris-Puffer, pH 8) mit $\alpha$-Chymotrypsin ($c_0$(CHY)=1 mg·ml$^{-1}$) in Abhängigkeit von der Auftragungsgeschwindigkeit;

Figur 5c: die Beladung von Bentonit $1_{500}$ (in 50 mM Citrat-Puffer, pH 4) mit humanem Serum Albumin ($c_0$ (HSA)=1 mg·ml$^{-1}$) in Abhängigkeit von der Auftragungsgeschwindigkeit.

Es wurden die folgenden Analysemethoden verwendet:

BET-Oberfläche/Porenvolumen nach BJH und BET:

Die Oberfläche und das Porenvolumen wurden mit einem vollautomatischen Stickstoffporosimeter der Firma Mikromeritics, Typ ASAP 2010 bestimmt.

Die Probe wird im Hochvakuum auf die Temperatur von flüssigem Stickstoff abgekühlt. Anschließend wird kontinuierlich Stickstoff in die Probenkammer dosiert. Durch die Erfassung der adsorbierten Gasmenge als Funktion des Druckes wird bei konstanter Temperatur eine Adsorptionsisotherme ermittelt. Nach einem Druckausgleich wird das Analysengas schrittweise entfernt und eine Desorptionsisotherme aufgenommen.

Zur Ermittlung der spezifischen Oberfläche und der Porosität nach der BET-Theorie werden die Daten gemäß DIN 66131 ausgewertet.

Das Porenvolumen wird ferner aus den Messdaten unter Anwendung der BJH-Methode ermittelt (E.P. Barret, L.G.Joiner, P.P. Haienda, J. Am. Chem. Soc. 73 (1951, 373). Bei diesem Verfahren werden auch Effekte der Kapillarkondensation berücksichtigt. Porenvolumina bestimmter Porengrößenbereiche werden durch Aufsummieren inkrementeller Porenvolumina bestimmt, die aus der Auswertung der Adsorptionsisotherme nach BJH erhalten werden. Das Gesamtporenvolumen nach der BJH-Methode bezieht sich auf Poren mit einem Durchmesser von 1,7 bis 300 nm.

Teilchengrößenbestimmung mittels dynamischer Lichtstreuung (Malvern)

Die Messungen werden mit einem Gerät "Mastersizer" der Firma Malvern Instruments Ltd., UK, entsprechend den Angaben des Herstellers durchgeführt. Die Messungen werden mit der vorgesehenen Probenkammer ("dry powder feeder") in Luft durchgeführt und die auf das Probenvolumen bezogenen Werte ermittelt.

Wassergehalt:

Der Wassergehalt der Produkte bei 105 ˚C wird unter Verwendung der Methode DIN/ISO-787/2 ermittelt.

Elementaranalyse:

Diese Analyse beruht auf dem Totalaufschluss der Tonmaterialien bzw. des entsprechenden Produktes. Nach dem Auflösen der Feststoffe werden die Einzelkomponenten mit herkömmlichen spezifischen Analysemethoden, wie z.B. ICP analysiert und quantifiziert.

Ionenaustauschkapazität:

Zur Bestimmung der Kationenaustauschkapazität wird das zu untersuchende Tonmaterial über einen Zeitraum von 2 Stunden bei 105 ˚C getrocknet. Danach wird das getrocknete Tonmaterial mit einem Überschuss an wässriger 2N $NH_4$Cl-Lösung 1 Stunde unter Rückfluss zur Reaktion gebracht. Nach einer Standzeit von 16 Stunden bei Raumtemperatur wird filtriert, worauf der Filterkuchen gewaschen, getrocknet und vermahlen wird und der $NH_4$-Gehalt im Tonmaterial durch Stickstoffbestimmung (CHN-Analysator "Vario EL III" der Firma Elementar in Hanau) nach den Herstellerangaben ermittelt. Der Anteil und die Art der ausgetauschten Metallionen wird im Filtrat durch ICP-Spektroskopie bestimmt.

Bestimmung des Sedimentvolumens:

Ein graduierter 100 ml Messzylinder wird mit 100 ml destilliertem Wasser gefüllt. 2 g der zu vermessenden Substanz werden langsam und portionsweise, je etwa 0,1 bis 0,2 g mit einem Spatel auf die Oberfläche des Wassers gegeben. Nach dem Absinken einer zugegebenen Portion wird die nächste Portion zugegeben. Nachdem die 2 g Substanz zugegeben und auf den Grund des Messzylinders abgesunken sind, wird der Zylinder für eine Stunde bei Raumtemperatur stehengelassen. Anschließend wird an der Graduierung des Messzylinders die Höhe des Sedimentvolumens in ml/2 g abgelesen. Für die Bestimmung des Sedimentvolumens nach 3-tägiger Lagerung in Wasser wird der Probenansatz mit Parafilm® verschlossen und für 3 Tage bei Raumtemperatur erschütterungsfrei stehen gelassen. Danach wird an der Graduierung des Messzylinders das Sedimentvolumen abgelesen.

Bestimmung des Montmorillonitgehalts über die Methylenblauadsorption

Der Methylenblauwert ist ein Maß für die innere Oberfläche der Tonmaterialien.

a) Herstellung einer Tetranatriumdiphosphat-Lösung

5,41 g Tetranatriumdiphosphat werden auf 0,001 g genau in einen 1000 ml Messkolben eingewogen und unter Schütteln bis zur Eichmarke mit dest. Wasser aufgefüllt.

b) Herstellung einer 0,5 %-igen Methylenblaulösung

In einem 2000 ml Becherglas werden 125 g Methylenblau in ca. 1500 ml dest. Wasser gelöst. Die Lösung wird abdekantiert und auf 25 l mit dest. Wasser aufgefüllt.

0,5 g feuchter Testbentonit mit bekannter innerer Oberfläche werden in einem Erlenmeyerkolben auf 0,001 g genau eingewogen. Es werden 50 ml Tetranatriumdiphosphatlösung zugegeben und die Mischung 5 Minuten zum Sieden erhitzt. Nach dem Abkühlen auf Raumtemperatur werden 10 ml 0,5 molare $H_2SO_4$ zugegeben und 80 bis 95 % des zu erwartenden Endverbrauchs an Methylenblaulösung zugegeben. Mit dem Glasstab wird ein Tropfen der Suspension aufgenommen und auf ein Filterpapier gegeben. Es bildet sich ein blau-schwarzer Fleck mit einem farblosen Hof. Es wird nun in Portionen von 1 ml weitere Methylenblaulösung zugegeben und die Tüpfelprobe wiederholt. Die Zugabe erfolgt solange, bis sich der Hof leicht hellblau färbt, also die zugegebene Methylenblaumenge nicht mehr vom Testbentonit absorbiert wird.

c) Prüfung von Tonmaterialien

Die Prüfung des Tonmaterials wird in der gleichen Weise durchgeführt wie für den Testbentonit. Aus der verbrauchten Menge an Methylenblaulösung lässt sich die innere Oberfläche des Tonmaterials berechnen.

381 mg Methylenblau/g Ton entsprechen nach diesem Verfahren einem Gehalt von 100 % Montmorillonit.

Bestimmung des Trockensiebrückstandes

Etwa 50 g des zu untersuchenden lufttrockenen Tonmaterials werden auf einem Sieb der entsprechenden Maschenweite eingewogen. Das Sieb wird an einen Staubsauger angeschlossen, der über ein unter dem Siebboden kreisenden Saugschlitz alle Anteile, die feiner als das Sieb sind, durch das Sieb heraussaugt. Das Sieb wird mit einem Plastikdeckel abgedeckt und der Staubsauger eingeschaltet. Nach 5 Minuten wird der Staubsauger abgeschaltet und die Menge der auf dem Sieb verbliebenen gröberen Anteile durch Differenzwägung ermittelt.

Bestimmung des Nasssiebrückstandes

Es wird zunächst eine 5 %-ige Suspension hergestellt, indem eine entsprechende Menge des zu untersuchenden Tonmaterials bei ca. 930 UpM ca. 5 Minuten in Wasser eingerührt wird. Die Suspension wird für weitere 15 Minuten bei ca. 1865 UpM gerührt und die Suspension dann durch ein Sieb der gewünschten Maschenweite gegossen. Der Rückstand wird so lange mit Leitungswasser gewaschen, bis das Waschwasser klar abläuft. Das Sieb mit dem Rückstand wird dann für 5 Minuten in ein Ultraschallbad gesetzt, um restliche Feinanteile zu entfernen. Der verbliebene Rückstand wird kurz mit Leitungswasser gewaschen und die Ultraschallbehandlung ggf. wiederholt, bis während der Ultraschallbehandlung keine Feinstoffe mehr in das Wasser übertreten. Das Sieb wird dann bis zur Gewichtskonstanz getrocknet. Zum Auswiegen wird der auf dem Sieb verbliebene Rückstand in eine gewogene Porzellanschale überführt.

Beispiel 1: Hitzebehandlung von Bentoniten

Für die Hitzebehandlung wurden zwei unterschiedliche Bentonite verwendet. Bentonit 1 ist ein natürlicher Calcium / Natriumbentonit. Bentonit 2 wurde durch Vermengen von Bentonit 1 mit 4,3 Gew.-% Soda, anschließendem Verkneten, Trocknen und Mahlen hergestellt. Die Bentonite wiesen einen Trockensiebrückstand von < 15 Gew.-% auf einem Sieb der Maschenweite 45 $\mu$m und einen Rückstand von < 7 Gew.-% auf einem Sieb der Maschenweite 75 $\mu$m. Die Eigenschaften der als Ausgangsmaterialien verwendeten Bentonite 1 und 2 sind in Tabelle 1 dargestellt.

Tabelle 1: Eigenschaften der als Ausgangsmaterialien verwendeten Bentonite 1 und 2

|  | Bentonit 1 | Bentonit 2 |
|---|---|---|
| Montmorillonitgehalt, bestimmt nach der Methylenblaumethode [%] | 75 | 78 |
| Kationenaustauschkapazität [meq/100 g] | 76 | 72 |
| Quellvolumen in destilliertem Wasser [ml/2 g] | 11 | > 15 |

Die als Ausgangsmaterial verwendeten Bentonite 1 und 2 wurden in einem Ofen für jeweils 1 Stunde auf die in Tabelle 2 angegebenen Temperaturen erhitzt. Die hitzebehandelten Bentonite 1 und 2 wurden aus dem Ofen herausgenommen und unter Luftzutritt auf Raumtemperatur abgekühlt. Danach wurden für die hitzebehandelten Bentonite jeweils das Quellvolumen bestimmt. Die gefundenen Werte sind in Tabelle 2 zusammengefasst.

Tabelle 2: Quellvolumina von thermisch behandelten und unbehandelten Bentoniten

| Behandlungstemperatur | Bentonit 1 Quellvolumen [ml/2 g] | Bentonit 2 Quellvolumen [ml/2 g] |
|---|---|---|
| Keine Behandlung | 11,0 | 17,0 |
| 300˚C | 12, 0 | 16,5 |
| 400˚C | 9,5 | 10,5 |
| 500˚C | 4,0 | 3,0 |

Durch die thermische Behandlung wird mit zunehmender Temperatur das Quellvermögen der Bentonite reduziert. Bei 500 ˚C entspricht das Quellvolumen der Schüttdichte des Bentonits in Wasser, also dem Sedimentvolumen. Bringt man Bentonite, welche bei diesen hohen Temperaturen behandelt wurden, in wässrige oder andere Flüssigkeiten ein, so zeigt sich auch bei langem Lagern kein Aufquellen der Bentonite mehr. Die typische Gelbildung des Bentonits kann nicht beobachtet werden. Die Partikel sinken schnell auf den Boden der Gefäße ab.

In Tabellen 3a, b sind die Eigenschaften eines unbehandelten Bentonits 1 einem bei 500 ˚C thermisch behandelten Bentonit $1_{500}$ gegenübergestellt.

Tabelle 3a: Physikalische Eigenschaften von thermisch behandelten und unbehandeltem Bentonit 1

| | Bentonit 1 | Bentonit $1_{500}$ |
|---|---|---|
| BET-Oberfläche [$m^2$/g] | 80 | 74,5 |
| Mikroporenoberfläche (BJH) [$m^2$/g] | 18 | 14 |
| Externe Oberfläche(BJH) [$m^2$/g] | 61 | 61 |
| Kumulatives Porenvolumen nach der BJH-Methode für Poren mit Durchmessern von 1,7 bis 300 nm, [$cm^3$/g] | 0,19 | 0,19 |
| Durchschnittsporendurchmesser [4V/A] nach BET | 8.9 | 9,3 |
| Durchschnittsporendurchmesser [4V/A] nach BJH | 11,1 | 11,5 |

Tabelle 3b: Siebrückstände des thermisch unbehandelten und behandelten Bentonits 1

| Bentonit | 1 | $1_{500}$ |
|---|---|---|
| Trockensiebrückstand auf 45 μm (Gew.-%) | 13,2 | 12,5 |
| Nasssiebrückstand auf: | | |
| 45 μm (Gew.-%) | 14,7 | 15,2 |
| 25 μm (Gew.-%) | 21,2 | 29 |

Ferner wurde die Kationenaustauschkapazität der thermisch behandelten und unbehandelten Bentonite 1 und 2 bestimmt. Die entsprechenden Werte sind in Tabelle 4 angegeben.

Tabelle 4: Kationenaustauschkapazität von thermisch behandelten und unbehandelten Bentoniten

| | Gesamt-Kationenaustauschkapazität in meq/100 g | Anteil der einwertigen Ionen in % der Gesamt-Kationenaustauschkapazität |
|---|---|---|
| Bentonit 1 | 70 | 20 |
| Bentonit $1_{500}$ (500˚C) | 65 | 20 |
| Bentonit 2 | 75 | 100 |
| Bentonit $2_{500}$ (500˚C) | 75 | 100 |

Beispiel 2: Entfernung von Restproteinen aus Weißwein

Das erfindungsgemäße Verfahren wurde zur Entfernung von Eiweiß aus Weißwein eingesetzt. Zur Analyse des Gehaltes an Restprotein wurde ein Verfahren aus dem Wein- und Bodenlabor Dr. Karl-Heinz Nilles, Volkach, DE, verwendet. Es wurden Dr. Nilles-Reagenz 1 (Blindwert) und Dr. Nilles-Reagenz 2 gemäß den Angaben und der Anleitung des Herstellers zur Ermittlung des Eiweißadsorptionsvermögens verwendet. Die Methode beruht auf einer Fällung des Weinproteins mit Hilfe eines eiweißspezifischen Reagenz mit anschließender photometrischer Trübungsmessung der farblosen Proteinfällung bei einer Wellenlänge von 623 nm.

Die thermisch nicht behandelten Bentonite 1 und 2 und die bei 500 °C behandelten Bentonite $1_{500}$ und $2_{500}$ wurden jeweils in Leitungswasser dispergiert und die Suspension zum Weißwein gegeben. Die Mengen betrugen ca. 50 g bis 200 g Tonmaterial/hl Wein. Nach einer Einwirkzeit von 15 Minuten wurde die Probe zur Abtrennung des Tonmaterials zentrifugiert und der klare Überstand abpipetiert. Der Eiweißgehalt im klaren Überstand wurde dann in der oben beschriebenen Weise mit dem Reagenz nach Dr. Nilles bestimmt.

Für die Untersuchungen wurden die folgenden Weine verwendet: 2002er Eschendorfer Lump, Silvaner trocken, Weingut Rainer Sauer, Eschendorf.

Die in Abhängigkeit von der zugegebenen Bentonitmenge ermittelten Extinktionen sind in Tabelle 5 angegeben.

Tabelle 5: Extinktionswerte in Abhängigkeit von der zugegebenen Bentonitmenge

| Dosierung (g/hl) | Bentonit 1 | Bentonit $1_{500}$ | Bentonit 2 | Bentonit $2_{500}$ |
|---|---|---|---|---|
| 25 | 0,195 | 0,240 | 0,131 | |
| 50 | 0,162 | 0,220 | 0,099 | |
| 75 | 0,148 | 0,190 | 0,051 | |
| 100 | 0,109 | 0,170 | 0,026 | |
| 125 | 0,079 | 0,143 | 0,008 | 0,203 |
| 150 | 0,072 | 0,112 | 0,009 | 0,188 |
| 175 | 0,054 | 0,094 | 0,007 | 0,178 |
| 200 | 0,046 | 0,074 | 0,006 | 0,158 |
| 225 | 0,036 | 0,060 | | 0,135 |
| 250 | 0,030 | 0,043 | | 0,117 |
| 275 | | 0,034 | 0,103 | |
| 300 | | 0,025 | 0,085 | |
| 325 | | 0,016 | 0,075 | |
| 350 | | 0,013 | 0,068 | |
| 375 | | 0,012 | 0,06 | |
| 400 | | 0,006 | 0,055 | |

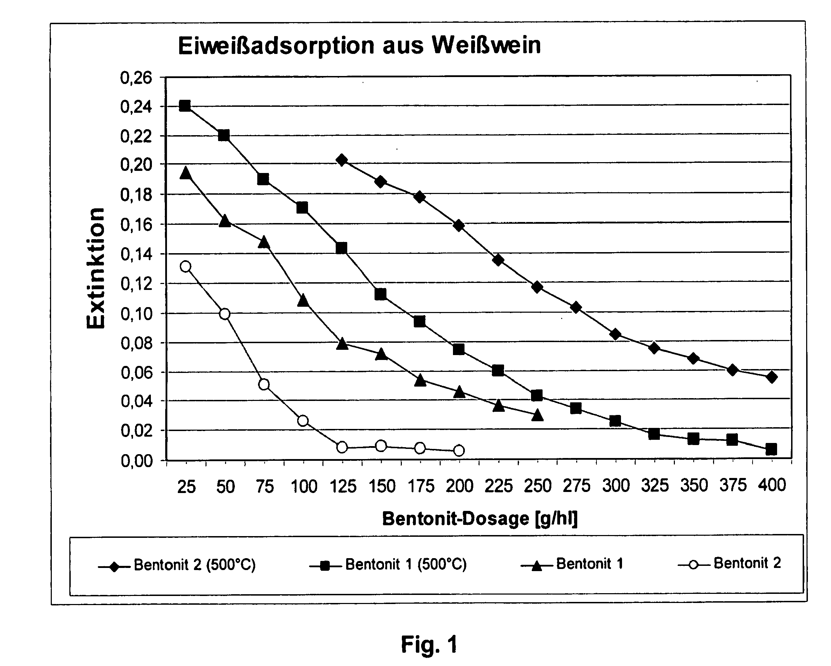

Um das Resteiweiß als Funktion der Bentonitzugabe grafisch darzustellen, wurde die Extinktion als Funktion der Bentonitzugabe aufgetragen. Ist alles Resteiweiß entfernt, so entsteht ein Plateau bei niedrigem Extinktionskoeffizienten, welcher der Geräteauflösung entspricht. Die entsprechende Grafik ist in Figur 1 dargestellt.

Figur 1 zeigt, dass durch die thermische Behandlung sowohl für Bentonit 1 als auch für Bentonit 2 die Eiweißadsorption aus Weißwein verringert wird, d.h. höhere Mengen an Bentonit benötigt werden, um eine äquivalente Trübung zu erreichen wie mit den unbehandelten Bentoniten 1 und 2. Für den Calcium-Bentonit 1 fällt der Unterschied in den für die gleiche Adsorption benötigten Mengen an Bentonit jedoch unerwartet gering aus. Um beispielsweise eine Trübung von 0,08 zu erreichen, werden beim unbehandelten Bentonit 1 etwa 125 g/hl benötigt und beim thermisch behandelten Bentonit $1_{500}$ etwa 190 g/hl. Anders verhält sich der durch Aktivierung mit Soda aus Bentonit 1 erhaltene Bentonit 2. Der thermisch unbehandelte Bentonit 2 zeigt eine sehr gute Proteinbindung in Folge vollständiger Delaminierung der Bentonitplättchen. Für eine Trübung von 0,8 wird eine Bentonitmenge von etwa 60 g/hl benötigt. Nach der thermischen Behandlung wird die Proteinbindung drastisch reduziert. Die Verringerung ist so stark, dass der thermisch behandelte Natriumbentonit $2_{500}$ das Weinprotein deutlich schlechter bindet als der thermisch unbehandelte Calciumbentonit 1 sowie der thermisch behandelte Calciumbentonit $1_{500}$. Um eine Trübung von 0,08 zu erreichen, wird beim

thermisch behandelten Bentonit $2_{500}$ eine Menge von etwa 310 g/hl benötigt.

Vergleicht man die Eigenschaften des thermisch behandelten Calciumbentonits $1_{500}$ mit den Eigenschaften des unbehandelten Natriumbentonits 2 zeigt sich zwar, dass für den Bentonit $1_{500}$ im Vergleich zum Bentonit 2 die etwa dreifache Menge zur Weinbehandlung nötig ist. Geht man jedoch von einem Batch-Verfahren weg zu einer Behandlung in Filterschichten oder -säulen, so weist der thermisch behandelte Calciumbentonit $1_{500}$ den Vorteil auf, praktisch nicht zu quellen. Dies ermöglicht es, Säulen zu packen, welche lediglich den thermisch behandelten Bentonit enthalten. Berechnet man die Proteinbindung auf eine Proteinbindung pro Volumen gepackte Säule um, ergibt sich für den thermisch behandelten Calciumbentonit $1_{500}$ eine wesentliche bessere Bindungskapazität pro Volumen, weil man den Natriumbentonit 2 in Folge seines hohen Quellvolumens etwa 1:9 mit nicht aktiven porösen Materialien, wie z.B. Zellulose oder Kieselgel etc., verdünnen müsste, um die durch das Quellen des Natriumbentonits verursachte Volumenzunahme in einer gepackten Säule ausgleichen zu können.

Beispiel 3: Bierfiltration

Für die Prüfung der Filtrierbarkeit von Bier wurde die Versuchsvorrichtung "Filtercheck®", Stabifix Brauereitechnik KG, Gräfelfing, DE, verwendet.

Die Versuchsvorrichtung "Filtercheck®", wurde nach Angaben des Herstellers gereinigt und zusammengesetzt. Anschließend wurde die Apparatur mittels Kühlbad und Umwälzpumpe auf eine Filtrationstemperatur von 0 ˚C temperiert.

500 ml Bier, aus welchem die Kohlensäure entfernt worden war, wurde in einer Bügelverschlussflasche auf 0 ˚C temperiert. Nachdem das Bier auf die Filtrationstemperatur abgekühlt worden war, wurden mittels eines Pulvertrichters 2,5 g Kieselgur (Filterzel®), John Menville, USA, Vertrieb in Deutschland über Lehrmann und Voss, Hamburg, Permeabilität (60 bis 70 mDarcy) sowie 50 g des zu untersuchenden Adsorptionsmittels zugegeben. Die Suspension wird anschließend ca. 60 Sekunden mit einem Magnetrührer homogenisiert und dann erneut auf 0 ˚C abgekühlt.

240 ml der Suspension wurden in die Versuchsvorrichtung überführt und diese anschließend verschlossen. Unter den Ablauf der Versuchsvorrichtung wurde ein Messzylinder gestellt. Der Ablauf wurde geöffnet und ein Druck von 1 bar an der Versuchsvorrichtung angelegt. Nachdem 40 ml Vorlauf abgelaufen waren, wurde der Messzylinder durch eine 180 ml Bügelverschlussflasche ersetzt und die Flasche mit filtriertem Bier gefüllt. Es wurde sowohl die Menge des filtrierten Biers bestimmt, als auch die Zeit, die für die Filtration benötigt wird. Das spezifische Filtratvolumen wurde gemäß der folgenden Formel berechnet und ist in hl/m$^2$h angegeben.

$$F_{sbez} = F \cdot V \cdot (t)^{-1/2}$$

V:  Gesamtfiltrationsvolumen (ml)
t:  Filtrationsdauer (s)
F:  Faktor (0,346 m$^{-2}$)

Die Eiweißentfernung aus dem Rohbier lässt sich durch die Abnahme der Trübung verfolgen. Die Messung der Trübung wurde mit dem Bierphotometer LTP 6B der Firma Dr. Lange bei einer Wellenlänge von 860 nm nach dem 90˚C-Zweistrahl-Streulichtverfahren durchgeführt.

Die Trübung ist in EBC-Einheiten angegeben. Die EBC-Skala basiert auf einer Formacin-Standardsuspension, die mit einer Genauigkeit von ± 2 % hergestellt werden kann (DIN 38404 C2). Dieses Konzentrat entspricht 100 EBC. Durch Verdünnung mit destilliertem Wasser können Eichlösungen mit beliebigen EBC-Werten hergestellt werden. Die Filtrationsversuche wurden mit den in Tabelle 6 angegebenen Adsorptionsmitteln durchgeführt:

Tabelle 6: Für die Bierfiltration verwendete Adsorptionsmittel

| Nr. | Adsorptionsmittel |
|---|---|
| 1 | 100% Bentonit $1_{500}$ |
| 2 | 50% hoch aufgeschlossene Bleicherde, 50% Kieselgel |
| 3 | 50% Bentonit $1_{500}$, 50% Kieselgel |

Vor dem Einsatz für die Bierfiltration wurde der Bentonit $1_{500}$ auf Teilchengrößen >45 μm abgesiebt, da ein hoher Anteil feiner Partikel erfahrungsgemäß zu einer schlechteren Durchlässigkeit der Schichten führt. Die eingesetzte

Probe von Bentonit $1_{500}$ wies einen Trockensiebrückstand von > 95 % auf einem Sieb der Maschenweite 45 $\mu$m und < 10 % auf einem Sieb der Maschenweite 75 $\mu$m auf. Der $D_{50}$ - Wert der Probe aus einer Malvern-Messung in Luft lag bei 60 $\mu$m.

Bezugsquellen:

Kieselgel: Zeochem C 560, Zeochem AG, CH;

Hochaufgeschlossene Bleicherde: Die Bleicherde wurde gemäß BT 1517888 B2 aus einem Rohton hergestellt, der 70 % Montmorillonit enthält. Sie wies eine BET-Oberfläche von 180 m$^2$/g sowie ein Schüttgewicht von 380 g/l auf. Das kumulative Porenvolumen von Poren mit einem Durchmesser von 1,7 bis 300 nm betrug 0,43 cm$^3$/g. Der pH-Wert, bestimmt durch Zugabe von 10 g Ton auf 100 ml destilliertes Wasser betrug 3.

Die Ergebnisse der Filtrationsversuche sind in Tabelle 7 zusammengefasst.

Ferner wurde die nicht-biologische Haltbarkeit des Bieres mit Hilfe des Forciertests ermittelt. Wird Bier auf 0 ˚C abgekühlt, kommt es zur Ausbildung einer Kältetrübung, d.h. ein Teil der Trübstoffe, vor allem Proteine und Harnstoffe, fällt aus. Diese Kältetrübung ist nur teilweise reversibel. Ein Ausfällen von Trübstoffen wird auch bei höheren Temperaturen beobachtet, wobei die Trübung um so schneller auftritt, je höher die Lagertemperatur ist. Kann die Trübung mit bloßem Auge wahrgenommen werden, ist die Grenze der nicht-biologischen Haltbarkeit erreicht.

Beim Forciertest wird das Bier künstlich gealtert, indem es bei 40 ˚C gelagert und in regelmäßigen Abständen auf 0 ˚C gekühlt wird. Die Ergebnisse hierzu am Anfang und nach zwei Zyklen (zwei Warmtage; WT) sind in den beiden letzten Spalten der Tabelle 7 angegeben.

Tabelle 7: Filtration von Rohbier

| Adsorptionsmittel | Filtrationsdaten | | | Forciertest 0/40˚C Trübung in EBC nach x WT | |
|---|---|---|---|---|---|
| | 220 ml (s) | $F_{spez}$ (hl/m$^2$/h) | EBC/15˚C Filtrat | 0 WT | 2 WT |
| 1 | 201 | 5,4 | 0,6 | 0,60 | 8,9 |
| 2 | 302 | 4,4 | 0,39 | 0,44 | 3,9 |
| 3 | 293 | 4,4 | 0,43 | 0,44 | 4,4 |

Die in Tabelle 7 angeführten Daten zeigen, dass die Mischung aus Kieselgel und thermisch behandeltem Tonmaterial vergleichbare Ergebnisse liefert, wie ein marktgängiges Produkt aus 50 % Kieselgel und 50 % hoch aufgeschlossener Bleicherde.

Beispiel 4: Untersuchung von weiteren Bentoniten

Analog zu Beispiel 1 wurden ein Bentonit 3 sowie ein Bentonit 4 für 1 Stunde bei 500 ˚C behandelt. Bentonit 4 wird aus dem Bentonit 3 durch eine stöchiometrische Aktivierung mit Soda erhalten. Bentonit 3 ist ein natürlicher Ca/Na-Bentonit.

Die Eigenschaften der Bentonite 3 und 4 sind in Tabelle 8 zusammengefasst. Die Quellvolumina für die thermisch unbehandelten und bei verschiedenen Temperaturen behandelten Bentonite 3 und 4 sind in Tabelle 9 zusammengefasst.

Tabelle 8: Eigenschaften der als Ausgangsmaterialien verwendeten Bentonite 3 und 4

| | Bentonit 3 | Bentonit 4 |
|---|---|---|
| Montmorillonitgehalt, bestimmt nach der Methylen-blaumethode [%] | 100 | 100 |
| Kationenaustauschkapazität [meq/100 g] | 105 | 105 |
| Quellvolumen in destilliertem Wasser [ml/2 g) | 12 - 18 | > 30 |
| Trockensiebrückstand<br>auf 45 $\mu$m | 11 | 15 |
| Nasssiebrückstand<br>auf 45 $\mu$m<br>auf 25 $\mu$m | 0,9<br>7,3 | 1,5<br>2,5 |

Tabelle 9: Quellvolumina (ml/2 g) verschiedener Bentonite 3 und 4

| Thermische Behandlung | Bentonit 3 | Bentonit 4 |
|---|---|---|
| Keine | 14,0 | 39,0 |
| 400 ˚C | 12,0 | 17,0 |
| 500 ˚C | 8,0 | 8,0 |

Die physikalischen Daten für den thermisch unbehandelten Bentonit 3 sowie den bei 500 ˚C behandelten Bentonit $3_{500}$ sind in Tabelle 10, die Daten für den thermisch unbehandelten Bentonit 4 sowie den bei 500 ˚C hitzebehandelten Bentonit $4_{500}$ sind in Tabelle 11 wiedergegeben.

Tabelle 10: Physikalische Eigenschaften von thermisch behandelten und unbehandeltem Bentonit 3

| | Bentonit 3 | Bentonit $3_{500}$ |
|---|---|---|
| BET-Oberfläche [$m^2$/g] | 80 | 74,5 |
| Mikroporenoberfläche [$m^2$/g] | 31 | 14 |
| Externe Oberfläche [$m^2$/g] | 49 | 61 |
| Kumulatives Porenvolumen nach BJH für Poren mit Durchmessern von 1,7 bis 300 nm, [$cm^3$/g] | 0,14 | 0,19 |
| Durchschnittsporendurchmesser [4V/A] nach BET | 7 | 9,3 |
| Durchschnittsporendurchmesser [4V/A] nach BJH | 10,3 | 11,5 |
| Gesamtkationen - Austausch-Kapazität [meq/100 g] | 105 | 74 |
| Anteil der einwertigen Ionen an der Gesamtkationen-Austauschkapazität [%] | 38 % | 37 % |

Tabelle 11: Physikalische Eigenschaften von thermisch behandeltem und unbehandeltem Bentonit 4

| | Bentonit 4 | Bentonit $4_{500}$ |
|---|---|---|
| BET-Oberfläche [$m^2$/g] | 24 | 19,5 |
| Mikroporenoberfläche [$m^2$/g] | 0 | 3,0 |
| Externe Oberfläche [$m^2$/g] | 8,1 | 16,5 |
| Kumulatives Porenvolumen nach BJH für Poren mit Durchmessern von 1,7 bis 300 nm, [$cm^3$/g] | 0,04 | 0,062 |
| Durchschnittsporendurchmesser [4V/A] nach BET [nm] | 18,7 | 11,9 |
| Durchschnittsporendurchmesser [4V/A] nach BJH [nm] | 13,0 | 14,0 |
| Gesamtkationen - Austausch-kapazität [meq/100 g] | 93 | 93 |
| Anteil der einwertigen Ionen [%] an der Gesamtionenaustauschkapazität | 100 % | 100 % |

Die Daten von Bentonit 3 und 4 zeigen, wie schon bei den Bentoniten 1 und 2 beobachtet, dass die Temperatur der Hitzebehandlung möglichst im Bereich von etwa 500 ˚C eingestellt werden sollte, um das Quellvermögen möglichst effizient zu unterdrücken. Wie schon bei den Bentoniten 1 und 2 gefunden wurde, zeigt auch hier der durch Austausch mit Natriumionen aktivierte Bentonit 4 nach der Temperaturbehandlung eine wesentlich niedrigere BET-Oberfläche als der natürliche Na-Ca-Bentonit 4. Analog sind die Daten beim kumulierten Porenvolumen.

Beispiel 5: Entfernung von Restproteinen aus Weißwein

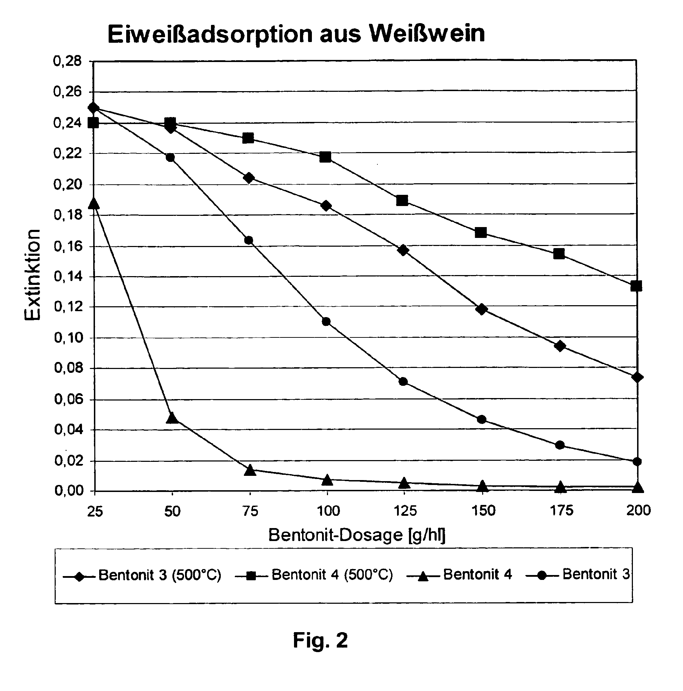

Die Bentonite 3 und $3_{500}$ sowie 4 und $4_{500}$ wurden analog Beispiel 2 für die Schönung von Weißwein eingesetzt. Die ermittelten Extinktionen sind in Tabelle 12 wiedergegeben. Die Extinktionen sind in Fig. 2 gegen die zugegebene Bentonitmenge aufgetragen.

Tabelle 12: Extinktionswerte in Abhängigkeit von der zugegebenen Bentonitmenge

| Menge (g/hl) | Bentonit 3 | Bentonit $3_{500}$ | Bentonit 4 | Bentonit $4_{500}$ |
|---|---|---|---|---|
| 25 | 0,250 | 0,250 | 0,188 | 0,240 |
| 50 | 0,217 | 0,237 | 0,048 | 0,240 |
| 75 | 0,163 | 0,204 | 0,014 | 0,230 |
| 100 | 0,110 | 0,186 | 0,007 | 0,217 |
| 125 | 0,071 | 0,157 | 0,005 | 0,189 |
| 150 | 0,046 | 0,118 | 0,003 | 0,168 |
| 175 | 0,029 | 0,094 | 0,002 | 0,154 |
| 200 | 0,018 | 0,074 | 0,002 | 0,133 |

Der hitzebehandelte Ca-Na-Bentonit $3_{500}$ zeigt eine wesentlich höhere Bindekapazität für Proteine, wie die niedrigere Extinktion des mit diesem Bentonit behandelten Weißweins gegenüber dem Vergleichsexperiment mit dem erhitzten Na-Bentonit $4_{500}$ zeigt.

Beispiel 6: Bindungseigenschaften von Modellproteinen

Die Adsorptionseigenschaften für den Bentonit $1_{500}$ in Bezug auf Modellproteine wurden sowohl in Lösung als auch in Säulenpackungen untersucht.. Hierzu wurde der hitzebehandelte Bentonit $1_{500}$ mit einem 45 $\mu$m Sieb abgesiebt. Eine solche Teilchengröße wurde eingestellt, um ein Verstopfen der Fritten der Säule mit feinteiligen Adsorbens zu verhindern. Das abgesiebte Material wies einen Trockensiebrückstand von > 95 % auf einem Sieb der Maschenweite 45 $\mu$m und 10 % auf einem Sieb der Maschenweite 75 $\mu$m auf, d.h. 85 % der Teilchen wiesen Größen von 45 $\mu$m - 75 $\mu$m auf.
Der $D_{50}$ - Wert der Probe aus einer Malvern-Messung in Luft lag bei 60 $\mu$m.
Für die Untersuchung der Adsorptionseigenschaften von hitzebehandelten Bentonit $1_{500}$ wurden die folgenden Modellproteine verwendet:

$\alpha$-Chymotrypsin (CHY)

$\alpha$-Chymotrypsin (E.C. 3.4.21.1) ist ein Verdauungsenzym, welches aus der Bauchspeicheldrüse von Rindern gewonnen wird. Das Enzym gehört zu den Proteasen und zur Untergruppe Serin-Hydrolasen. Das Enzym besitzt eine Masse von 25,3 kDa. Der isoelektrische Punkt liegt zwischen pH 8,1 und 8,6.

Humanes Serumalbumin (HSA)

HSA ist ein globoläres Transportprotein, das als Carrier für Fettsäuren und Amphiphile aus dem Blut ins umgebende Gewebe dient. Es besitzt ein Gesamtgewicht von 66,3 kDa. Der isoelektrische Punkt des Moleküls liegt bei pH 4,9.

Alkalische Phosphatase (AP)

Alkalische Phosphatase (E.C. 3.1.3.1) ist eine unspezifische Phosphorsäuremonoesterase, die in allen Spezies von E. coli bis zum Menschen vorkommt. AP ist ein Metalloprotein, das zwei $Zn^{2+}$ und ein $Mg^{2+}$ Ion enthält. Die in den Beispielen verwendete AP wird aus Darmschleimhaut von Kälbern isoliert. Der isoelektrische Punkt liegt bei pH 6,0. Das Molekülgewicht des Dimers beträgt 140 kDa.

β-Glucanase (BGL)

1,3-β-Glucanase und 1,4-β-Glucanase (E.C. 3.2.1.6) katalysieren die hydrolytische 1,3- bzw. 1,4-Spaltung von β-D-Glucanen. Das Molekülgewicht des BGL-HIS beträgt 26,7 kDa. Sein isoelektrischer Punkt liegt bei pH 6,1.

Für die Untersuchungen wurde der thermisch behandelte Bentonit $1_{500}$ verwendet. Das Material wurde durch Absieben auf eine Teilchengröße von > 45 $\mu$m eingestellt.

Der $D_{50}$ - Wert der Probe aus einer Malvern-Messung in Luft lag bei 60 $\mu$m.

Probenvorbereitung für Adsorptionsversuche im statischen System

Von den zu untersuchenden Bentoniten werden je 25,0 mg in 50 ml-Reaktionsgefäße eingewogen. Die Tonmaterialien werden in 10 ml des entsprechenden Probenvorbereitungspuffers suspendiert und 30 Minuten im Ultraschallbad behandelt. Anschließend werden die Proben für 1 Stunde auf einem Schütteltisch bei Raumtemperatur und 100 Upm geschüttelt. Die Proben werden bei 4000 g für 10 Minuten zentrifugiert und anschließend der Überstand abpipetiert. Das erhaltene Pellet wird in 10 ml bidest. $H_2O$ resuspendiert, anschließend für 5 Minuten bei 100 Upm geschüttelt und die Probe dann bei 4000 g für 10 Minuten zentrifugiert. Der Überstand wird abpipettiert und die erhaltenen Pellets 16 Stunden bei 60 ˚C getrocknet.

Probenvorbereitung für Adsorptionsversuche im dynamischen System

100 mg des Tonmaterials Bentonit $1_{500}$ werden in einem 1 ml-Eppendorfhütchen mit 1 ml des entsprechenden 50 mM Puffers aufgeschlämmt und in eine mit einem Stempel nach unten abgeschlossene Säule pipetiert. Das zweite Säulenendstück wird aufgesetzt und die Säule so mit der FPLC-Anlage verbunden, dass sie von der mobilen Phase von unten nach oben durchströmt wird. Die FPLC-Pumpe wird auf eine Flussrate eingeregelt, für welche die Kapazität des Tonmaterials ermittelt werden soll. Der bewegliche Stempel der Säule wird während der Equilibrierung mit 20 ml 50 mM Puffern langsam handfest angezogen und anschließend um eine Viertel Gewindedrehung entlastet.

Untersuchung der pH-Abhängigkeit der Proteinadsorption

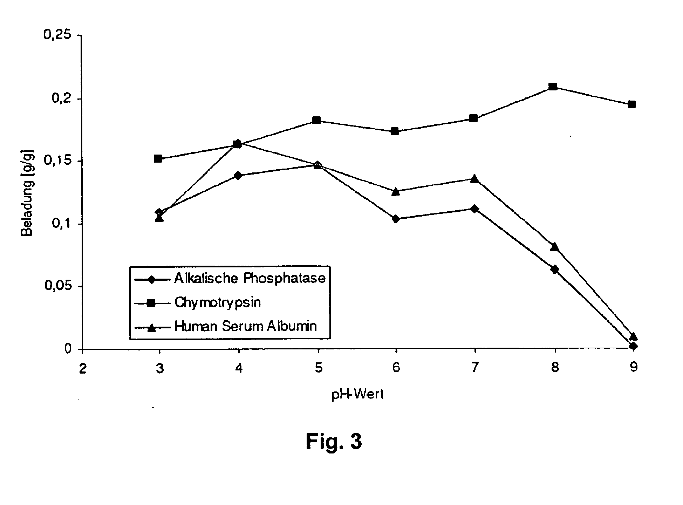

25 mg des wie oben angegeben equilibrierten Tonmaterials Bentonit $1_{500}$ wurden in ein 25 ml-Falconröhrchen überführt. Von den Modellproteinen wurden jeweils Stammlösungen mit einer Konzentration von 2 mg/ml in bidest. $H_2O$ hergestellt. Die Proteinlösungen wurden im Verhältnis 1:1 mit 100 mM Puffern der pH-Werte 3 bis 8 zu einem Gesamtvolumen von 20 ml verdünnt und auf die Tonmaterialien gegeben. Von den Modellproteinen wurden Standardreihen in 50 mM Puffern der entsprechenden pH-Werte hergestellt. Die Proben und die Standards wurden für 3 Stunden bei 4 ˚C und 100 Upm auf einem Schütteltisch inkubiert. Nach beendeter Inkubation wurden die Proben für 10 Minuten bei 4000 g zentrifugiert. Die Konzentration der Modellproteine in den Probenüberständen wurde photometrisch bei 280 nm gegen die Standardreihe des entsprechenden Proteins im jeweiligen Puffer bestimmt. Die Beladung des Bentonits errechnet sich aus der Differenz der Proteinkonzentration vor und nach der Inkubation, sowie der Masse des Tonmaterials und dem Volumen der Proteinlösung. Die Ergebnisse der Messungen sind als Mittelwerte einer Doppelbestimmung in Figur 3 aufgetragen.

Beim thermisch behandelten Bentonit $1_{500}$ findet die maximale Beladung bei einem pH-Wert statt, der eine bzw. für α-Chymotrypsin eine halbe Einheit unterhalb des isoelektrischen Punktes des Proteins liegt.

Aufnahme von Proteinadsorptionsisothermen

Zur Aufnahme von Adsorptionsisothermen wurde die Abhängigkeit der Gleichgewichtsbeladung des thermisch behandelten Bentonit $1_{500}$ von der Konzentration des Modellproteins im Überstand der Probe untersucht.

Die Versuche wurden für die einzelnen Proteine jeweils bei den pH-Werten und in den Puffern vorgenommen, für welche das Maximum der Adsorption ermittelt worden war. Dazu wurde eine Proteinstammlösung mit einem Proteingehalt von 3 mg/ml in 50 mM Puffer hergestellt und nach der in Tabelle 13 angegebenen Vorschrift mit dem jeweiligen Puffer verdünnt. Diese Lösung wurde dann jeweils zu 25 mg des wie oben beschrieben equilibrierten Bentonits $1_{500}$ gegeben.

Tabelle 13: Herstellung der Proteinlösungen

| Nr. | Volumen Proteinstammlösung (ml) | Volumen Puffer (ml) | $C_{protein}$ (mg/ml) | Massenverhältnis Protein/ Absorber |
|---|---|---|---|---|
| 1 | 2 | 33 | 0,17 | 0,24 |
| 2 | 5 | 30 | 0,43 | 0,60 |
| 3 | 8 | 27 | 0,69 | 0,96 |
| 4 | 12 | 23 | 1,03 | 1,44 |
| 5 | 16 | 19 | 1,37 | 1,92 |
| 6 | 20 | 15 | 1,71 | 2,40 |
| 7 | 24 | 11 | 2,06 | 2,88 |

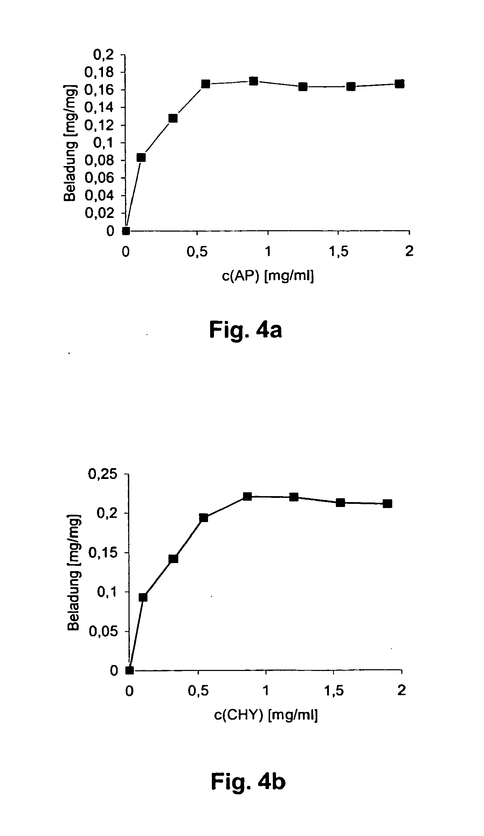

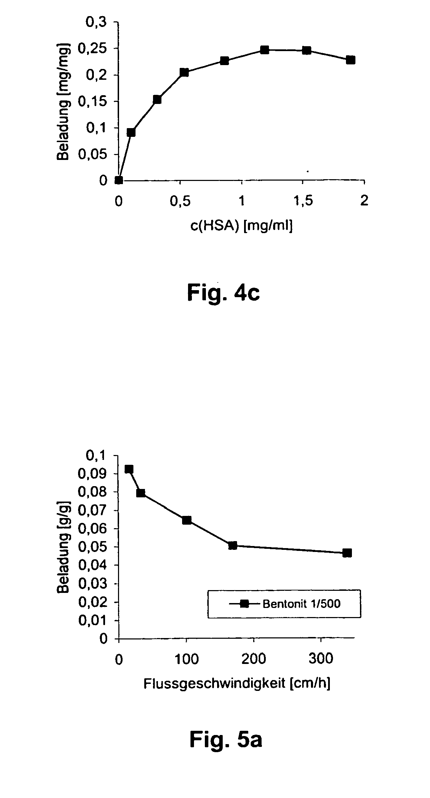

Für jedes Modellprotein wurde eine Standardreihe im entsprechenden Puffer angesetzt. Die mit dem Bentonit $1_{500}$ behandelten Proben und die Standardlösungen wurden für 3 Stunden bei 4 ˚C und 100 Upm auf einem Schütteltisch inkubiert und anschließend für 10 Minuten bei 4000 g zentrifugiert. Die Beladung des Bentonits wurde durch die photometrische Quantifizierung des Proteins im Überstand ermittelt und ergibt sich aus der Differenz der Konzentration des Proteins vor und nach Behandlung mit dem Bentonit $1_{500}$. Die Messwerte sind als Mittelwerte einer Doppelbestimmung in Figur 4 dargestellt.

Bestimmung der Proteinbindungskapazitäten im dynamischen System

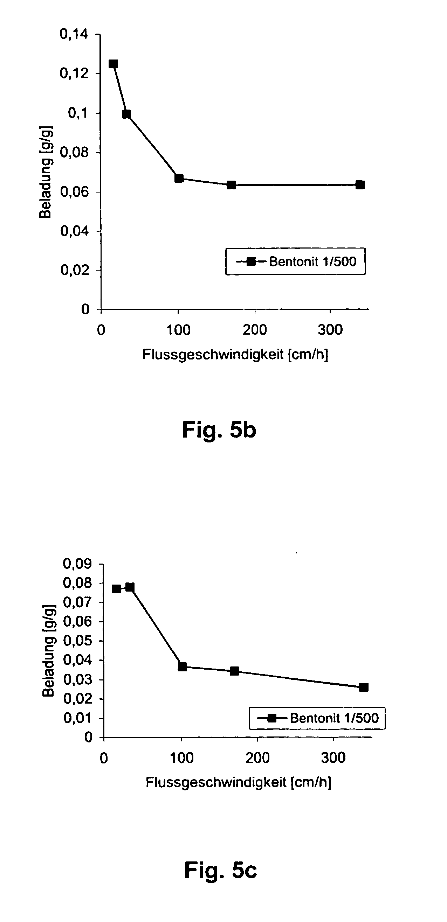

Zur Untersuchung der Beladung des thermisch modifizierten Bentonits $1_{500}$ wurde zunächst wie oben beschrieben 25 ml einer Lösung des jeweiligen Proteins in 50 mM Puffer bei variablen Flussraten durch eine mit 100 mg des Bentonits $1_{500}$ gepackte Säule gepumpt. Die Versuche wurden jeweils in Puffern der pH-Werte durchgeführt, für welche die maximale Adsorption im statischen System ermittelt wurde. Der Proteingehalt im Durchlauf wurde photometrisch bei 280 nm gegen eine Standardreihe des Proteins im entsprechenden Puffer ermittelt. Die flussratenabhängigen Proteinbeladungen sind in Figur 5 als Mittelwerte einer Doppelbestimmung dargestellt.

In Tabelle 14 sind die pH-Werte zusammengefasst, bei welchen die maximale Beladung des thermisch modifizierten Bentonits $1_{500}$ für die untersuchten Modellproteine erreicht wird.

Tabelle 14: pH-Werte für die maximale Beladung des thermisch modifizierten Bentonits $1_{500}$ mit Modellproteinen

| Protein | pH-Wert der maximalen Beladung | pI-Wert des Proteins |
|---|---|---|
| Alkalische Phosphatase | 5 | 6,0 |
| Humanes Serumalbumin | 4 | 4,9 |
| α-Chymotrypsin | 8 | 8,1 - 8,6 |

Die in Tabelle 14 aufgeführten Werte zeigen, dass für den thermisch modifizierten Bentonit $1_{500}$ die maximale Beladung bei einem pH-Wert erreicht wird, welcher etwa eine Einheit unterhalb des pI-Wertes des entsprechenden Modellproteins liegt.

Interpretation der Protein-Adsorptionsisothermen

Aus dem Verlauf der Adsorptionsisotherme lässt sich die maximale Kapazität des verwendeten, thermisch modifizierten Tonmaterials für die einzelnen Proteine aus dem Teil der Isotherme abschätzen, für den die Beladung mit steigender Konzentration einen konstanten Wert annimmt. In Tabelle 15 sind diese abgeschätzten Kapazitäten angegeben.

Tabelle 15: Abschätzung maximaler Kapazität des Bentonits $1_{500}$ für verschiedene Modellproteine

| Protein | Bentonit $1_{500}$ (g/g) |
|---|---|
| Alkalische Phosphortase | 0,17 |
| Humanes Serumalbumin | 0,25 |

(fortgesetzt)

| Protein | Bentonit $1_{500}$ (g/g) |
|---|---|
| $\alpha$-Chymotrypsin | 0,22 |

Proteinbindungskapazitäten im dynamischen System

Der thermisch modifizierte Bentonit $1_{500}$ wurde bei einer Korngrößenverteilung von > 45 $\mu$m in Chromatographiesäulen gepackt und bei verschiedenen Flussgeschwindigkeiten von 16,8 bis 339,6 cm/h mit verschiedenen Modellproteinen beladen. Die für die verschiedenen Modellproteine ermittelten Beladungen sind in Tabelle 16 für verschiedene Flussgeschwindigkeiten angegeben.

Tabelle 16: Beladung des thermisch modifizierten Bentonits $1_{500}$ mit Modellproteinen bei verschiedenen Flussgeschwindigkeiten

| Flussrate [cm/h] | Beladung mit alkalischer Phosphatase [$\mu$g/mg] | Beladung mit $\alpha$-Chymotrypsin [$\mu$g/mg] | Beladung mit humanem Serumalbumin [$\mu$g/mg] |
|---|---|---|---|
| 16,8 | 93 | 125 | 77 |
| 34,2 | 79 | 99 | 78 |
| 102,0 | 64 | 67 | 36 |
| 169,8 | 50 | 63 | 34 |
| 339,6 | 46 | 63 | 26 |

**Patentansprüche**

1. Verfahren zur Abtrennung von Proteinen aus flüssigen Medien, wobei:

    - ein proteinhaltiges flüssiges Medium bereitgestellt wird;
    - das proteinhaltige flüssige Medium zur Abreicherung an Proteinen mit einem hitzebehandelten Tonmaterial in Kontakt gebracht wird, das eine Quellfähigkeit in Wasser nach einer Stunde von weniger als 15 ml/2 g aufweist, und
    - das an Proteinen abgereicherte flüssige Medium von dem Tonmaterial abgetrennt wird.

2. Verfahren nach Anspruch 1, wobei das hitzebehandelte Tonmaterial erhalten wird, indem:

    - ein Rohtonmaterial bereitgestellt wird, und
    - das Rohtonmaterial für eine Dauer von zumindest 10 Minuten auf eine Temperatur von zumindest 400 ˚C erhitzt wird.

3. Verfahren nach einem der vorhergehenden Ansprüche, wobei das hitzebehandelte Tonmaterial eine Ionenaustauschkapazität von zumindest 15, bevorzugt zumindest 40, besonders bevorzugt zumindest 65 meq/100 g aufweist.

4. Verfahren nach einem der vorhergehenden Ansprüche, wobei das hitzebehandelte Tonmaterial ein Verhältnis von austauschbaren zweiwertigen Kationen zu austauschbaren einwertigen Kationen von zumindest 4 : 1 aufweist.

5. Verfahren nach einem der vorhergehenden Ansprüche, wobei das hitzebehandelte Tonmaterial einen Anteil an zweiwertigen Kationen an der Ionenaustauschkapazität von zumindest 50 % aufweist.

6. Verfahren nach einem der vorhergehenden Ansprüche, wobei das hitzebehandelte Tonmaterial eine spezifische Oberfläche von weniger als 300 m$^2$/g, bevorzugt weniger als 200 m$^2$/g, besonders bevorzugt weniger als 120 m$^2$/g aufweist.

7. Verfahren nach einem der vorhergehenden Ansprüche, wobei das hitzebehandelte Tonmaterial nach einer Lagerung in Wasser für 3 Tage ein Sedimentvolumen von weniger als 15, bevorzugt weniger als 10 ml/g aufweist.

**8.** Verfahren nach einem der vorhergehenden Ansprüche, wobei das hitzebehandelte Tonmaterial keiner Oberflächenaktivierung durch Säure unterworfen worden ist.

**9.** Verfahren nach einem der vorhergehenden Ansprüche, wobei das hitzebehandelte Tonmaterial vor der Aufgabe des flüssigen Mediums auf einen pH-Wert von 3,5 bis 6,0 equilibriert wird.

**10.** Verfahren nach einem der vorhergehenden Ansprüche, wobei das flüssige Medium zur Abtrennung der Proteine durch eine Filterpackung geleitet wird, welche zumindest anteilig aus dem hitzebehandelten Tonmaterial gebildet ist.

**11.** Verfahren nach einem der vorhergehenden Ansprüche, wobei das flüssige Medium zur Abtrennung der Proteine über eine Chromatographiesäule geleitet wird, deren Packung im wesentlichen aus dem hitzebehandelten Tonmaterial gebildet ist.

**12.** Verfahren nach einem der vorhergehenden Ansprüche, wobei nach der Abtrennung des gereinigten flüssigen Mediums die an das Tonmaterial gebundenen Proteine mit einem Elutionsmittel eluiert werden.

**13.** Verfahren nach Anspruch 12, wobei das Elutionsmittel einen zum flüssigen Medium unterschiedlichen pH-Wert aufweist.

**14.** Verfahren nach einem der vorhergehenden Ansprüche, wobei das Tonmaterial im Gemisch mit einem weiteren Adsorptionsmaterial verwendet wird.

**15.** Verfahren nach Anspruch 14, wobei das weitere Adsorptionsmaterial ausgewählt ist aus der Gruppe, die gebildet ist aus Kieselgel, Zellulose und Polyvinylpyrrolidon.

**16.** Verfahren nach Anspruch 14 oder 15, wobei das Tonmaterial und das weitere Adsorptionsmaterial, bezogen auf das Gewicht, in einem Verhältnis zwischen 1:10 und 10:1 vorliegen.

**Claims**

**1.** Method for separating proteins from liquid media, wherein:

- a protein-containing liquid medium is provided;
- the protein-containing liquid medium is brought into contact, for protein depletion, with a heat-treated clay material that has a swelling capacity in water after an hour of less than 15 ml/2 g, and
- the protein-depleted liquid medium is separated from the clay material.

**2.** Method according to claim 1, wherein the heat-treated clay material is obtained by:

- providing a crude clay product, and
- heating the crude clay material for a period of at least 10 minutes to a temperature of at least 400 ˚C.

**3.** Method according to one of the previous claims, wherein the heat-treated clay material has an ion-exchange capacity of at least 15, preferably at least 40, particularly preferably at least 65 meq/100 g.

**4.** Method according to one of the previous claims, wherein the heat-treated clay material has a ratio of exchangeable divalent cations to exchangeable monovalent cations of at least 4 : 1.

**5.** Method according to one of the previous claims, wherein the heat-treated clay material has a proportion of divalent cations to the ion-exchange capacity of at least 50 %.

**6.** Method according to one of the previous claims, wherein the heat-treated clay material has a specific surface area of less than 300 $m^2$/g, preferably less than 200 $m^2$/g, particularly preferably less than 120 $m^2$/g.

**7.** Method according to one of the previous claims, wherein the heat-treated clay material has a sediment volume of less than 15, preferably less than 10 ml/g after storage in water for 3 days.

**8.** Method according to one of the previous claims, wherein the heat-treated clay material has not been subjected to surface activation by acid.

**9.** Method according to one of the previous claims, wherein the heat-treated clay material is equilibrated to a pH of from 3.5 to 6.0 before the charging of the liquid medium.

**10.** Method according to one of the previous claims, wherein the liquid medium is passed, for the separation of the proteins, through a filter pack at least a proportion of which is formed by the heat-treated clay material.

**11.** Method according to one of the previous claims, wherein the liquid medium is passed, for the separation of the proteins, over a chomatography column the packing of which is formed substantially from the heat-treated clay material.

**12.** Method according to one of the previous claims, wherein after the separation of the purified liquid medium the proteins bound to the clay material are eluted with an eluent.

**13.** Method according to claim 12, wherein the eluent has a different pH from the liquid medium.

**14.** Method according to one of the previous claims, wherein the clay material is used mixed with a further adsorption material.

**15.** Method according to claim 14, wherein the further adsorption material is selected from the group which is formed by silica gel, cellulose and polyvinylpyrrolidone.

**16.** Method according to claim 14 or 15, wherein the clay material and the further adsorption material are present in a ratio of between 1:10 and 10:1, based on weight.

**Revendications**

**1.** Procédé de séparation de protéines à partir de milieux liquides,

- un milieu liquide contenant des protéines étant mis à disposition
- pour l'appauvrissement en protéines, le milieu liquide contenant des protéines étant amené en contact avec une matière argileuse modifiée thermiquement, qui après une heure présente une capacité de gonflement inférieure à 15 ml/2g, et
- le milieu liquide appauvri en protéines étant séparé de la matière argileuse.

**2.** Procédé selon la revendication 1, la matière argileuse étant obtenue en ce que :

- une matière argileuse brute est mise à disposition, et
- la matière argileuse brute est chauffée pour une durée minimale de 10 minutes à une température d'au moins 400˚C.

**3.** Procédé selon l'une quelconque des revendications précédentes, la matière argileuse modifiée thermiquement présentant une capacité d'échange d'ions d'au moins 15, de préférence d'au moins 40, de façon particulièrement préférée d'au moins 65 meq/100 g.

**4.** Procédé selon l'une quelconque des revendications précédentes, la matière argileuse modifiée thermiquement présentant un rapport de cations bivalents échangeables aux cations monovalents échangeables d'au moins 4 : 1.

**5.** Procédé selon l'une quelconque des revendications précédentes, la matière argileuse modifiée thermiquement présentant une part de cations bivalents par rapport à la capacité d'échanges d'ions d'au moins 50 %.

**6.** Procédé selon l'une quelconque des revendications précédentes, la matière argileuse modifiée thermiquement présentant une surface spécifique inférieure à 300 m$^2$/g, de préférence inférieure à 200 m$^2$/g, de façon particulièrement préférée inférieure à 120 m$^2$/g.

**7.** Procédé selon l'une quelconque des revendications précédentes, la matière argileuse modifiée thermiquement présentant après un stockage dans l'eau de 3 jours un volume de sédiments inférieur à 15, de préférence inférieur à 10 ml/g.

**8.** Procédé selon l'une quelconque des revendications précédentes, la matière argileuse modifiée thermiquement n'ayant été soumise à aucune activation de la surface par acide.

**9.** Procédé selon l'une quelconque des revendications précédentes, la matière argileuse modifiée thermiquement étant équilibrée à une valeur pH de 3,5 à 6,0, avant la charge du milieu liquide.

**10.** Procédé selon l'une quelconque des revendications précédentes, pour la séparation des protéines, le milieu liquide étant dirigé à travers un paquet de filtres, qui est constitué au moins proportionnellement de la matière argileuse modifiée thermiquement.

**11.** Procédé selon l'une quelconque des revendications précédentes, pour la séparation des protéines, le milieu liquide étant dirigé par l'intermédiaire d'une colonne de chromatographie, dont le paquet est constitué en majeure partie de la matière argileuse modifiée thermiquement.

**12.** Procédé selon l'une quelconque des revendications précédentes, après la séparation du milieu liquide purifié, la protéine liée à la matière argileuse étant éluée à l'aide d'un produit d'élution.

**13.** Procédé selon la revendication 12, le produit d'élution présentant une valeur pH différente de celle du milieu liquide.

**14.** Procédé selon l'une quelconque des revendications précédentes, la matière argileuse étant utilisée en mélange avec une autre matière d'adsorption.

**15.** Procédé selon la revendication 14, l'autre matière d'adsorption étant choisie dans le groupe formé par le gel de silice, la cellulose et la poly-vinyl pyrrolidone.

**16.** Procédé selon la revendication 14 ou 15, la matière argileuse et l'autre matière d'adsorption étant présentes dans un rapport au poids compris entre 1 : 10 et 10 : 1.

Fig. 1

Fig. 2

**Fig. 3**

**Fig. 4a**

**Fig. 4b**

**Fig. 4c**

**Fig. 5a**

**Fig. 5b**

**Fig. 5c**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 1160812
- DE 1717084 A
- DE 1717085 B
- DE OS1517888 A
- DE 1642767 B

### In der Beschreibung aufgeführte Nicht-Patentliteratur

- **C. Buttersack ; K. Nowikow ; A. Schaper ; K. Buchholz.** *Zuckerind,* 1994, vol. 119 (4), 284-291
- **E.P. Barret ; L.G.Joiner ; P.P. Haienda.** *J. Am. Chem. Soc.,* 1951, vol. 73, 373